(19) **Europäisches Patentamt**

**European Patent Office**

**Office européen des brevets**

(11) **EP 0 579 640 B1**

(12) **EUROPEAN PATENT SPECIFICATION**

(45) Date of publication and mention
of the grant of the patent:
**24.07.2002 Bulletin 2002/30**

(51) Int Cl.[7]: **C07K 14/475**, C07K 16/22

(21) Application number: **92907539.8**

(86) International application number:
**PCT/GB92/00595**

(22) Date of filing: **03.04.1992**

(87) International publication number:
**WO 92/18627 (29.10.1992 Gazette 1992/27)**

(54) **GLIAL MITOGENIC FACTORS, THEIR PREPARATION AND USE**

GLIALMITOGENE FAKTOREN, IHRE HERSTELLUNG UND VERWENDUNG

FACTEURS MITOGENIQUES GLIAUX, LEUR PREPARATION ET LEUR UTILISATION

(84) Designated Contracting States:
**AT BE CH DE DK ES FR GB GR IT LI LU MC NL SE**

(30) Priority: **10.04.1991 GB 9107566**

(43) Date of publication of application:
**26.01.1994 Bulletin 1994/04**

(73) Proprietors:
• **LUDWIG INSTITUTE FOR CANCER RESEARCH**
**Norfolk Place, Paddington, London W2 1PG (GB)**
• **CAMBRIDGE NEUROSCIENCE RESEARCH, INC.**
**Cambridge, MA 02139 (US)**

(72) Inventors:
• **GOODEARL, Andrew, David, John**
**Charleywood Hertfordshire WD3 5PY (GB)**
• **WATERFIELD, Michael, Derek**
**Newbury Berkshire RG13 1RN (GB)**
• **STROOBANT, Paul**
**London N8 9AS (GB)**
• **MINGHETTI, Luisa**
**I-48012 Bagnacavallo (IT)**
• **MARCHIONNI, Mark, Andrew**
**Arlington, MA 02174 (US)**

(74) Representative: **Bizley, Richard Edward et al**
**Hepworth, Lawrence, Bryer & Bizley**
**Merlin House**
**Falconry Court**
**Baker's Lane**
**Epping Essex CM16 5DQ (GB)**

(56) References cited:
• **The Journal of Neuroscience, vol. 4, no. 1, January 1984, (Washington, DC, US), G.E. LEMKE et al.: "Identification and purification of glial growth factor", pages 75-83, see the whole article (cited in the application)**
• **Methods in Enzymology, vol. 147, 1987, (Peptide Growth Factors, part B, edited by D. BARNES et al.), Academic Press, Inc., (New York, US), J.P. BROKES: "Assay and isolation of glial growth factor from the bovine pituitary", pages 217-225, see the whole document (cited in the application)**
• **The Journal of Cell Biology, vol. 110, no. 4, April 1990, (New York, US), J.B. DAVIS et al.: "Platelet-derived growth factors and fibroblast growth factors are mitogens for rat schwann cells", pages 1353-1360, see the whole document (cited in the application)**
• **World Patents Index, week 7123, class B04, AN=71-40056S, Derwent Publications Ltd, London, GB, & JP,A,46020832 (TEIKOKU HORMONE MANUF. LTD), see the abstract**
• **The Journal of Biological Chemistry, vol. 255, no. 18, 25 September 1990, (US), J.P. BROCKES et al.: "Purification and preliminary characterization of a glial growth factor from the bovine pituitary", pages 8374-8377, see the whole article (cited in the application)**
• **Nature, vol. 348, no. 6298, 15 November 1990, (London, GB), H. KIMURA et al.: "Structure, expression and function of a schwannoma-derived growth factor", pages 257-260, see the whole article (cited in the application)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

This invention relates to new polypeptides found in vertebrate species, which polypeptides are mitogenic growth factors, e.g. having activity on cultured Schwann cells. The invention is also concerned, inter alia, with a novel isolation process capable of producing such factors, and the therapeutic application of such factors. The invention further includes useful peptides characteristic of such factors.

The glial cells of vertebrates constitute the specialized connective tissue of the central and peripheral nervous systems. Important glial cells include Schwann cells which provide myelin sheathing around the axons of neurones, thereby forming individual nerve fibres. Schwann cells provide a sheath effect by forming concentric layers of membrane around adjacent neurone axons, twisting as they develop around the axons. These myelin sheaths are a susceptible element of many nerve fibres, and damage to Schwann cells, or failure in growth and development, can be associated with significant demyelination or nerve degeneration characteristic of a number of peripheral nervous system diseases and disorders. In the development of the nervous system, it has become apparent that cells require various factors to regulate their division and growth, and various such factors have been identified in recent years, including some found to have an effect on Schwann cell division or development.

Thus, Brockes et al., inter alia, in J. Neuroscience, 4 (1984), No 1, 75-83 describe a protein growth factor present in extracts from bovine brain and pituitary tissue, which was named Glial Growth Factor (GGF). This factor stimulated cultured rat Schwann cells to divide against a background medium containing ten percent foetal calf serum. The factor was also described as having a molecular weight of 31,000 and as readily dimerizing. In Meth. Enz., 147 (1987), 217-225, Brockes describes a Schwann cell-based assay for the factor, and emphasises that in purification of the factor the use of reversed-phase HPLC using trifluoroacetic acid as an ion pairing agent must be avoided if biological activity is to be retained.

The J. Neuroscience article of Brockes et al., supra, describes methods of purification of GGF to apparent homogeneity. In brief, one large-scale purification method described involves extraction of the lyophilized bovine anterior lobes and chromatography of material obtained thereby using NaCl gradient elution from CM-cellulose. Gel filtration is then carried out with an Ultrogel column, followed by elution from a phosphocellulose column, and finally, small-scale SDS gel electrophoresis. Alternatively, the CM-cellulose material was applied directly to a phosphocellulose column, fractions from the column were pooled and purified by preparative native gel electrophoresis, followed by a final SDS gel electrophoresis.

Brockes et al. observe that in previously reported gel filtration experiments (Brockes et al., J. Biol. Chem. 255 (1980) 8374-8377), the major peak of growth factor activity was observed to migrate with a molecular weight of 56,000, whereas in the first of the above-described procedures activity was predominantly observed at molecular weight 31,000. It is reported that the GGF dimer is largely removed as a result of the gradient elution from CM-cellulose in this procedure.

In PNAS, 82 (1985), 3930-3934, Benveniste et al. describe a T lymphocyte-derived glial growth promoting factor. This factor, under reducing conditions, exhibits a change in apparent molecular weight on SDS gels.

In Nature, 348 (1990), 257-260, Kimura et al. describe a factor they term Schwannoma-derived growth factor (SDGF) obtained from a sciatic nerve sheath tumour. The authors state that SDGF does not stimulate the incorporation of tritium-labelled TdR into cultured Schwann cells under conditions where, in contrast, partially purified pituitary fraction containing GGF is active. SDGF has an apparent molecular weight of between 31,000 and 35,000.

In J. Cell. Biol., 110 (1990), 1353-1360, Davis et al. describe the screening of a number of candidate mitogens. Rat Schwann cells were used, the chosen candidate substances being examined for their ability to stimulate DNA synthesis in the Schwann cells in the present of 10% FCS (foetal calf serum), with and without forskolin. One of the factors tested was GGF-carboxymethyl cellulose fraction (GGF-CM), which was mitogenic in the presence of FCS, with and without forskolin. The work revealed that in the presence of forskolin, inter alia, platelet derived growth factor (PDGF) was a potent mitogen for Schwann cells, PDGF having previously been thought to have no effect on Schwann cells.

One aspect of the present invention is new glial growth factors which are distinguished from known factors, including the above, which factors are mitogenic for Schwann cells against a background of foetal calf plasma (FCP). The invention also provides processes for the preparation of these factors, and an improved method for defining activity of these and other factors.

Therapeutic application of the factors is a further significant aspect of the invention.

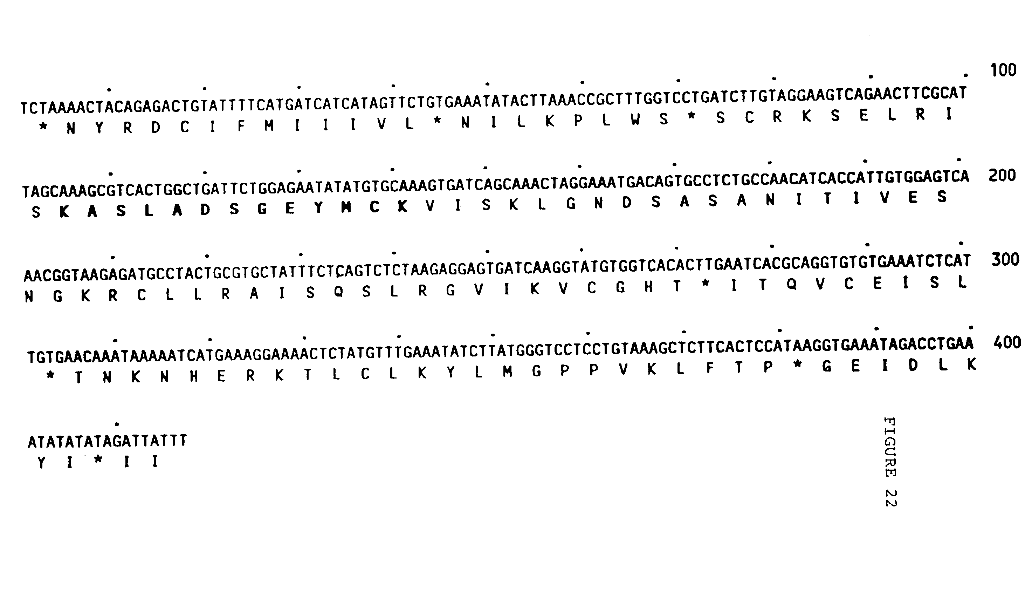

Accordingly, the present invention provides a basic polypeptide factor or portion thereof obtainable by hydroxylapatite HPLC and having mitogenic activity stimulating the division of Schwann cells in the presence of foetal calf plasma, and when isolated using reversed-phase HPLC retaining at least 50% of said activity after 10 weeks incubation in 0.1% trifluoroacetic acid at 4°C, said basic polypeptide factor having a molecular weight of from 30 kD to 36 kD, and encoded by a DNA sequence shown in Figure 22.

Included within the scope of the invention is a basic polypeptide factor having mitogenic activity stimulating the division of Schwann cells in the presence of foetal calf plasma, a molecular weight of from about 30 kD to about 36 kD, and including within its amino acid sequence any one or more of the following peptide sequences:

F K G D A H T E

A S L A D E Y E Y M X K

T E T S S G L X L K

A S L A D E Y E Y M R K

A G Y F A E X A R

T T E M A S E Q G A

A K E A L A A L K

F V L Q A K K

E T Q P D P G Q I L K K V P M V I G A Y T

E Y K C L K F K W F K K A T V M

E X K F Y V P

K L E F L X A K; and

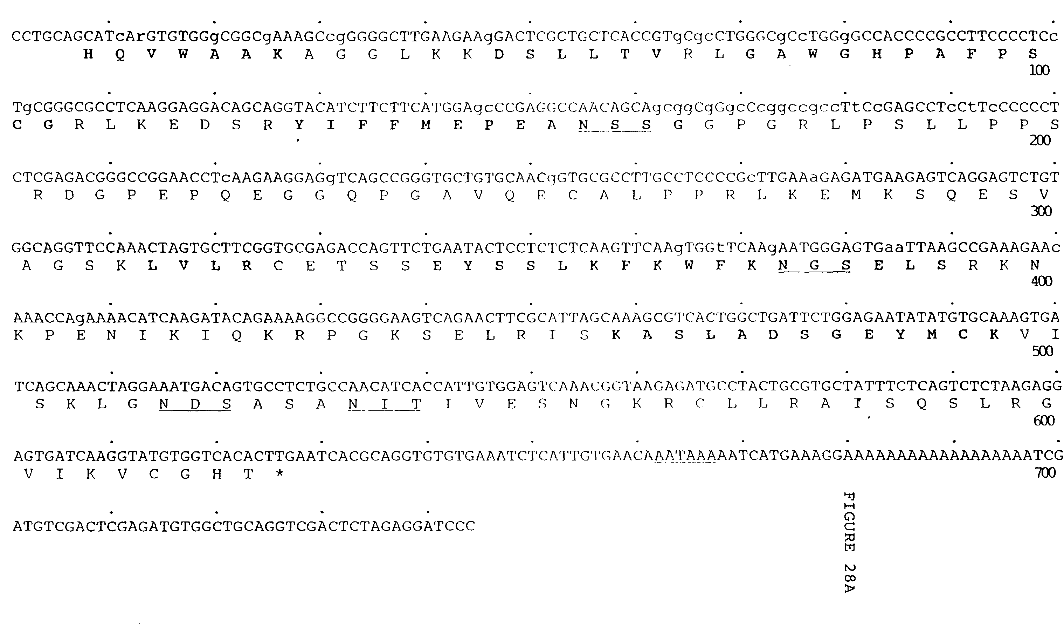

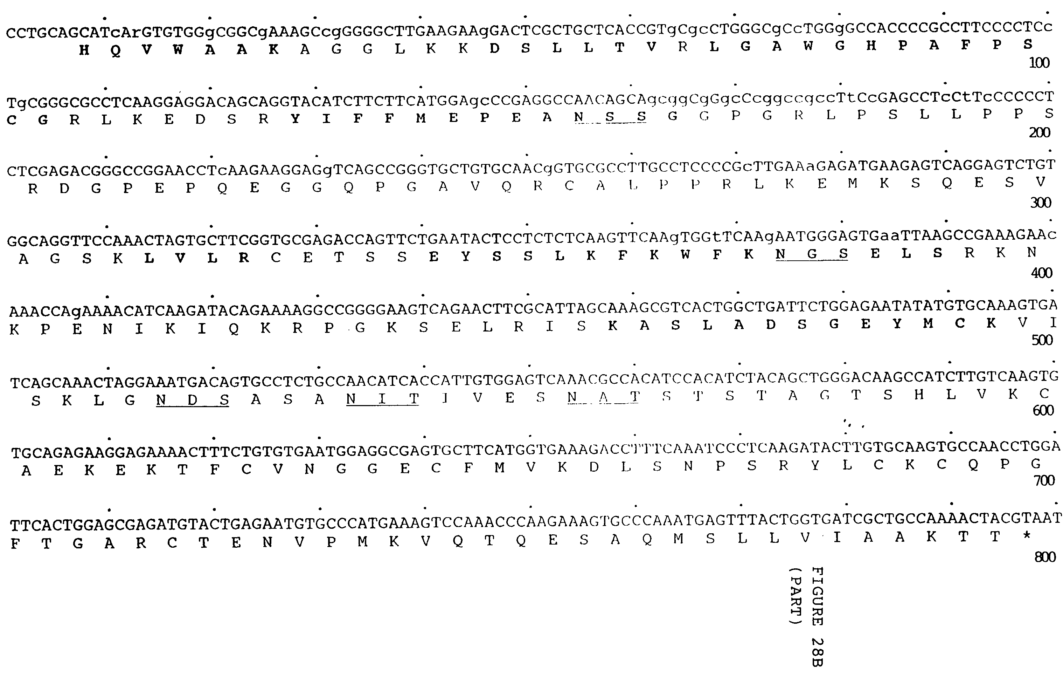

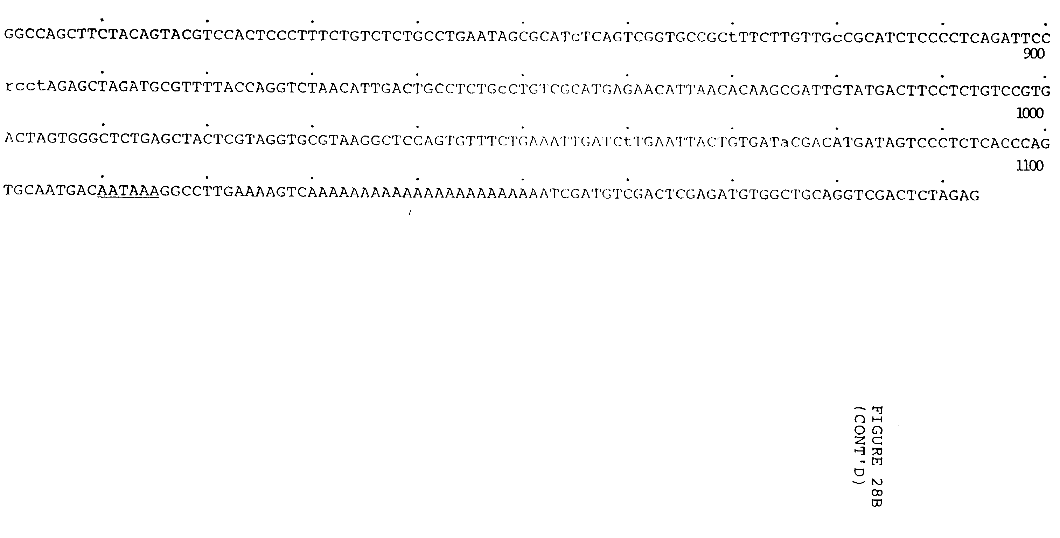

The invention also provides a basic polypeptide factor or portion thereof obtainable by hydroxylapatite HPLC and stimulating the division of Schwann cells in the presence of foetal calf plasma, and when isolated using reversed-phase HPLC retaining at least 50% of said activity after 10 weeks incubation in 0.1% trifluoroacetic acid at 4°C, said basic polypeptide factor having a molecular weight of from 55 kD to 63 kD, and encoded by a DNA sequence as shown in any of Figures 28a, 28b or 28c; or the DNA sequence represented by nucleotides 281-557 of the sequence shown in figure 28a.

Included within the scope of the invention is a basic polypeptide factor having mitogenic activity stimulating the division of Schwann cells in the presence of foetal calf plasma, a molecular weight of from about 55 kD to about 63 kD, and including within its amino acid sequence any one or more of the following peptide sequences:

V H Q V W A A K

Y I F F M E P E A X S S G

L G A W G P P A F P V X Y

W F V V I E G K

A S P V S V G S V Q E L V Q R

V C L L T V A A P T

K V H Q V W A A K

K A S L A D S G E Y M X K

D L L L X V

The peptide sequences set out above, 12 derived from the smaller molecular weight polypeptide factor, and 9 from the larger molecular weight polypeptide factor may be useful, inter alia, as probe sources for polypeptide factors of the invention, for investigating, isolating or preparing such factors (or corresponding gene sequences) from a range of different species, or preparing such factors by recombinant technology, and in the generation of corresponding antibodies, by conventional technologies, preferably monoclonal antibodies, which are themselves useful investigative tools in relation to the present factors and are possible medicaments.

The availability of short peptides from the highly purified factors of the invention has enabled (as will be seen hereinafter) additional sequence to be determined.

Thus, the invention further embraces a polypeptide factor having glial cell mitogenic activity and including an amino acid sequence encoded by:-

(a) a DNA sequence shown in any one of Figures 28a, 28b or 28c;

(b) a DNA sequence shown in Figure 22;

(c) the DNA sequence represented by nucleotides 281-557 of the sequence shown in Figure 28a; or

(d) a DNA sequence hybridizable to any one of the DNA sequences according to (a), (b) or (c).

Whilst the present invention is not limited to a particular set of hybridization conditions, the following protocol gives general guidance which may, if desired, be followed:-

Thus, DNA probes may be labelled to high specific activity (approximately $10^8$ to $10^9$ $^{32}$P.dmp/µg) by nick translation or by PCR reactions according to Schowalter and Sourer (Anal. Biochem., 177, 90-94, 1989) and purified by desalting on G-150 Sephadex columns. Probes may be denatured (10 minutes in boiling water followed by immersion into ice water), then added to hybridization solutions of 80% buffer B (2g polyvinylpyrolidine, 2g Ficoll-400, 2g bovine serum albumim, 50ml 1M Tris HCL(pH 7.5) 58g NaCl, 1g sodium pyrophosphate, 10g sodium dodecyl sulfate, 950ml $H_2O$) containing 10% dextran sulfate at $10^6$ dpm $^{32}$P per ml and incubated overnight (say, 16 hours) at 60°C. The filters may then be washed at 60°C, first in buffer for 15 minutes followed by three 20-minute washes in 2X SSC, 0.1% SDS then one for 20 minutes in 1x SSC, 0.1% SDS.

In other respects, polypeptides of the invention include:

(a) a basic polypeptide factor which has, if obtained from bovine pituitary material, an observed molecular weight, whether in reducing conditions or not, of from about 30 kD to about 36 kD on SDS-polyacrylamide gel electrophoresis using the following molecular weight standards:

| | |
|---|---|
| Lysozyme (hen egg white) | 14,400 |
| Soybean trypsin inhibitor | 21,500 |
| Carbonic anhydrase (bovine) | 31,000 |
| Ovalbumin (hen egg white) | 45,000 |
| Bovine serum albumin | 66,200 |
| Phosphorylase B (rabbit muscle) | 97,400; |

which factor has mitogenic activity stimulating the division of rat Schwann cells in the presence of foetal calf plasma, and when isolated using reversed-phase HPLC retains at least 50% of said activity after 10 weeks incubation in 0.1% trifluoroacetic acid at 4°C; and

(b) a basic polypeptide factor which has, if obtained from bovine pituitary material, an observed molecular weight, under non-reducing conditions, of from about 55 kD to about 63 kD on SDS-polyacrylamide gel electrophoresis using the following molecular weight standards:

| | |
|---|---|
| Lysozyme (hen egg white) | 14,400 |
| Soybean trypsin inhibitor | 21,500 |
| Carbonic anhydrase (bovine) | 31,000 |
| Ovalbumin (hen egg white) | 45,000 |
| Bovine serum albumin | 66,200 |
| Phosphorylase B (rabbit muscle) | 97,400; |

which factor has mitogenic activity stimulating the division of rat Schwann cells in the presence of foetal calf plasma, and when isolated using reversed-phase HPLC retains at least 50% of said activity after 4 days incubation in 0.1% trifluoroacetic acid at 4°C.

For convenience of description only, the lower molecular weight and higher molecular weight factors of this invention are referred to hereinafter as "GGF-I" and "GGF-II", respectively.

It will be appreciated that the molecular weight range limits quoted are not exact, but are subject to slight variation depending, inter alia, upon the source of the particular polypeptide factor. A variation of, say, about 10% would not, for example, be impossible for material from another source.

Another important aspect of the invention is an isolated DNA having a sequence encoding a polypeptide having glial cell mitogenic activity and comprising:-

(a) a DNA sequence shown in any one of Figures 28a, 28b or 28c;

(b) a DNA-sequence shown in Figure 22;

(c) the DNA sequence represented by nucleotides 281-557 of the sequence shown in Figure 28a; or

(d) a DNA sequence hybridizable to any one of the DNA sequences according to (a), (b) or (c).

The invention includes any modifications or equivalents of the above two polypeptide factors which do not exhibit a significantly reduced activity. For example, modifications in which amino acid content or sequence is altered without substantially adversely affecting activity are included. By way of illustration, in EP-A-109748 muteins of native proteins are disclosed in which the possibility of unwanted -S-S- bonding is avoided by replacing any cysteine in the native sequence not necessary for biological activity with a neutral amino acid. The statements of effect and use contained herein are therefore to be construed accordingly, with such uses and effects employing modified or equivalent factors as aforesaid being part of the invention.

The new sequences of the invention open up the benefits of recombinant technology. The invention thus also includes the following aspects:-

(a) a DNA construct comprising a DNA sequence as defined above and in operable reading frame position in a vector under the control of a control sequence permitting expression of said sequence in chosen host cells after transformation thereof by said construct (preferably said control sequence includes a regulatable promotor, e.g. Trp) - it will be appreciated that the selection of a promotor and regulatory sequences (if any) are matters of choice for those of skill in the art;

(b) host cells modified by incorporating a construct as defined in (a) immediately above so that said DNA sequence may be expressed in said host cells - the choice of host is not critical, and chosen cells may be prokaryotic or eukaryotic and may be genetically modified to incorporate said construct by methods known in the art; and

(c) a process for preparation of a glial cell mitogenic factor as defined above comprising cultivating said modified host cells under conditions permitting expression of said DNA sequence, which conditions can be readily determined, for any particular embodiment, by those of skill in the art of recombinant DNA technology. Glial cell mitogens prepared by this means are included in the present invention.

None of the factors described in the art has the combination of characteristics possessed by the present new polypeptide factors.

As indicated, the Schwann cell assay in part used to characterise the present factors employs a background of foetal calf plasma. In all other respects, the assay can by the same as that described by Brockes et al. in Meth. Enz., supra, but with 10% FCP replacing 10% FCS. This difference in assay technique is significant, since the absence of platelet-derived factors in foetal calf plasma (as opposed to serum) enables a more rigorous definition of activity on Schwann cells to be provided by eliminating potentially spurious effects from some other factors.

Yet a further aspect of the invention is a process for the preparation of a polypeptide as defined above, comprising extracting vertebrate brain material optionally pituitary material e.g bovine pituitary to obtain protein, subjecting the resulting extract to chromatographic purification comprising hydroxylapetite HPLC and thereafter to SDS-polyacrylamide gel electrophoresis and collecting that fraction therefrom which has an observed molecular weight of about 30 kD to 36 kD and/or that fraction which has an observed molecular weight of about 55 kD to 63 kD if, in either case, subjected to SDS-polyacrylamide gel electrophoresis using the following molecular weight standards:

| Lysozyme (hen egg white) | 14,400 |
| Soybean trypsin inhibitor | 21,500 |
| Carbonic anhydrase (bovine) | 31,000 |

(continued)

| Ovalbumin (hen egg white) | 45,000 |
| Bovine serum albumin | 66,200 |
| Phosphorylase B (rabbit muscle) | 97,400; |

in the case of the aforesaid smaller molecular weight fraction whether in reducing conditions or not, and in the case of the aforesaid larger molecular weight fraction under non-reducing conditions, and which fraction(s) exhibit(s) activity stimulating the division of rat Schwann cells against a background of foetal calf plasma.

Preferably, the chromatographic purification includes a step wherein the protein initially extracted from brain material is first subjected to carboxymethyl cellulose chromatography, and/or also including that after hydroxylapatite HPLC, cation exchange chromatography, gel filtration, and/or reversed-phase HPLC be employed prior to the SDS-Polyacrylamide gel electrophoresis. At each stage in the process, activity may be determined using Schwann cell incorporation of radioactive iododeoxyuridine as a measure in an assay generally as described by Brockes in Meth. Enz., supra, but modified by substituting 10% FCP for 10% FCS. As already noted, such as assay is an aspect of the invention in its own right when applied generally to the assay of any substance for CNS or PNS cell, eg Schwann cell, mitogenic effects.

An assay for glial cell mitogenic activity in which a background of foetal calf plasma may be employed against which to assess DNA synthesis in glail cells stimulated (if at all) by a substance under assay.

Another aspect of the invention is a pharmaceutical or veterinary formulation comprising any factor as defined above formulated for pharmaceutical or veterinary use, respectively, optionally together with an acceptable diluent, carrier or excipient and/or in unit dosage form.

In using the factors of the invention, conventional pharmaceutical or veterinary practice may be employed to provide suitable formulations or compositions.

Thus, the formulations of this invention can be applied to parenteral administration, for example, intravenous, subcutaneous, intramuscular, intraorbital, ophthalmic, intraventricular, intracranial, intracapsular, intraspinal, intracisternal, intraperitoneal, topical, intranasal, aerosol, scarification, and also oral, buccal, rectal or vaginal administration.

Parenteral formulations may be in the form of liquid solutions or suspensions; for oral administration, formulations may be in the form of tablets or capsules; and for intranasal formulations, in the form of powders, nasal drops, or aerosols.

Methods well known in the art for making formulations are to be found in, for example, "Remington's Pharmaceutical Sciences". Formulations for parenteral administration may, for example, contain as excipients sterile water or saline, polyalkylene glycols such as polyethylene glycol, oils of vegetable origin, or hydrogenated naphthalenes. Biocompatible, biodegradable lactide polymer, lactide/glycolide copolymer, or polyoxyethylene-polyoxypropylene copolymers may be used to control the release of the present factors. Other potentially useful parenteral delivery systems for the factors include ethylene-vinyl acetate copolymer particles, osmotic pumps, implantable infusion systems, and liposomes. Formulations for inhalation may contain as excipients, for example, lactose, or may be aqueous solutions containing, for example, polyoxyethylene-9-lauryl ether, glycocholate and deoxycholate, or may be oily solutions for administration in the form of nasal drops, or as a gel to be applied intranasally. Formulations for parenteral administration may also include glycocholate for buccal administration, methoxysalicylate for rectal administration, or citric acid for vaginal administration.

The present factors can be used as the sole active agents or can be used in combination with other active ingredients, eg, other growth factors which could facilitate neuronal survival in neurological diseases, or peptidase or protease inhibitors.

The concentration of the present factors in the formulations of the invention will vary depending upon a number of issues, including the dosage to be administered, and the route of administration.

In general terms, the factors of this invention may be provided in an aqueous physiological buffer solution containing about 0.1 to 10% w/v compound for parenteral administration. General dose ranges are from about 1 $\mu$g/kg to about 1 g/kg of body weight per day; a preferred dose range is from about 0.01 mg/kg to 100 mg/kg of body weight per day. The preferred dosage to be administered is likely to depend upon the type and extent of progression of the pathophysiological condition being addressed, the overall health of the patient, the make up of the formulation, and the route of administration.

As indicated above, Schwann cells (the glial cells of the peripheral nervous system) are stimulated to divide in the presence of the factors of the invention. Schwann cells of the peripheral nervous system are involved in creating the myelin sheath around individual nerve fibres which is important for proper conduction of electrical impulses to muscles and from sensory receptors.

There are a variety of peripheral neuropathies in which Schwann cells and nerve fibres are damaged, either primarily or secondarily. There are many neuropathies of both sensory and motor fibres (Adams and Victor, Principles of Neurology). The most important of those neuropathies are probably the neuropathies associates with diabetes, Landry-Guillain-Barre syndrome, neuropathies caused by carcinomas, and neuropathies caused by toxic agents (some of which are used to treat carcinomas).

The invention, however, envisages treatment or prophylaxis of conditions where nervous system damage has been brought about by any basic cause, eg infection or injury. Thus, in addition to use of the present factors in the treatment of disorders or diseases of the nervous system where demyelination or loss of Schwann cells is present, such glial growth factors can be valuable in the treatment of disorders of the nervous system that have been caused by damage to the peripheral nerves.

Following damage to peripheral nerves, the regeneration process is led by the growth or the re-establishment of Schwann cells, followed by the advancement of the nerve fibre back to its target. By speeding up the division of Schwann cells one could promote the regenerative process following damage.

Furthermore, there are a variety of tumours of glial cells the most common of which is probably neurofibromatosis, which is a patchy small tumour created by overgrowth of glial cells. Also, it has been found that an activity very much like GGF can be found in some Schwann cell tumours, and therefore an inhibitors of the action of the present factors on their receptors provides a therapy for glial tumours. The invention thus specifically includes a method for the prophylaxis or treatment of a glial tumour, which comprises administering an effective amount of a substance which inhibits the binding of a factor as defined above to a receptor therefor.

In general, the invention includes the use of present polypeptide factors in the prophylaxis or treatment of any pathophysiological condition of the nervous system in which a factor-sensitive or factor-responsive cell type is involved.

The polypeptide factors of the invention can also be used as immunogens for making antibodies, such as monoclonal antibodies, following standard techniques.

Accordingly the invention also provides an antibody to the polypeptides as hereinbefore described and such antibodies are included within the present invention. These antibodies can, in turn, be used for diagnostic purposes. Thus, conditions perhaps associated with abnormal levels of the factor may be tracked by using such antibodies. In vitro techniques can be used, employing assays on isolated samples using standard methods. Imaging methods can also be employed in which the antibodies are, for example, tagged with radioactive isotopes which can be imaged outside the body using techniques employed in the art of, for example, tumour imaging.

Another aspect of the invention is the use of a polypeptide as hereinbefore described or a corresponding nucleic acid sequence, in investigating, isolating or preparing a glial cell mitogen or a gene sequence corresponding thereto, or in generating a corresponding antibody.

The invention also includes the general use of the present factors as glial cell mitogens in vivo or in vitro, and the factors for such use. One specific embodiment is thus a method for producing a glial cell mitogenic effect in a vertebrate by administering an effective amount of a factor of the invention. A preferred embodiment is such a method in the treatment or prophylaxis of a nervous system disease or disorder.

A further general aspect of the invention is the use of a factor of the invention in the manufacture of a medicament, for the treatment of a nervous disease or disorder or for neural regeneration or repair.

Also included in the invention are the use of the factors of the invention in competitive assays to identify or quantify molecules having receptor binding characteristics corresponding to those of said polypeptides. The polypeptides may be labelled, optionally with a radioisotope. A competitive assay can identify both antagonists and agonists of the relevant receptor.

In another aspect, the invention provides the use of each one of the factors of the invention in an affinity isolation process, optionally affinity chromatography, for the separation of a respective corresponding receptor. Such processes for the isolation of receptors corresponding to particular proteins are known in the art, and a number of techniques are available and can be applied to the factors of the present invention. For example, in relation to IL-6 and IFN-gamma the reader is referred to Novick, D. et al., J. Chromatogr.; 1990, June 27; 510, 331-7, in relation to gonadotropin releasing hormone reference is made to Hazum, E., J. Chromatogr.; 1990, June 27; 510, 233-8, in relation to G-CSF reference is made to Fukunaga, R., et al., J. Biol. Chem.; 1990, Aug. 15; 265(23), 14008-15, in relation to vasoactive intestinal peptide reference is made to Couvineau, A., et al., J. Biol. Chem.; 1990, Aug. 5; 265(22), 13386-90, in relation to IL-2 reference is made to Smart, J.E., et al., J. Invest. Dermatol.; 1990, June; 94(6 Suppl.), 158S-163S, and in relation to human IFN-gamma reference is made to Stefanos, S., et al., J. Interferon Res.; 1989, Dec., 9(6), 719-30.

The following Examples are not intended to limit the invention, but usefully illustrate the same, and provide specific guidance for effective preparative techniques.

As will be seen from Example 3 hereinafter, the present factors exhibit mitogenic activity on a range of cell types. The activity in relation to fibroblasts indicates a wound repair ability, and the invention encompasses this use. The general statements of invention above in relation to formulations and/or medicaments and their manufacture should clearly be construed to include appropriate products and uses. This is clearly a reasonable expectation for the present invention having regard to, inter alia, reports of similar activities for FGFs. Reference can be made, for example, to Sporn et al., "Peptide Growth Factors and their Receptors I", page 396 (Baird and Bohlen) in the section headed "FGFs in Wound Healing and Tissue Repair".

In the accompanying drawings:-

Figures 1 to 8 relate to Example 1 hereinafter, and are briefly described below:

Figure 1 is the profile for product from carboxymethyl cellulose chromatography;

Figure 2 is the profile for product from hydroxylapatite HPLC;

Figure 3 is the profile for product from Mono S FPLC;

Figure 4 is the profile for product from Gel filtration FPLC;

Figures 5 and 6 are the profiles for the two partially purified polypeptide products from reversed-phase HPLC; and

Figures 7 and 8 are dose response curves for the GGF-I and GGF-II fractions from reversed-phase HPLC using either a foetal calf serum or a foetal calf plasma background;

Figures 9 to 12 show peptides derived from GGF-I and GGF-II (see Example 2 hereinafter), Figures 10 and 12 specifically showing novel sequences:

In Figure 10, Panel A, the sequences are shown of GGF-I peptides used to design degenerate oligonucleotide probes and degenerate PCR primers. Some of those sequences in Panel A were also used to design synthetic peptides. Panel B shows the novel peptides that were too short (less than 6 amino acids) for the design of degenerate probes or degenerate PCR primers;

In Figure 12, Panel A, the sequences are shown of GGF-II peptides used to design degenerate oligonucleotide probes and degenerate PCR primers. Some of these sequences in Panel A were also used to design synthetic peptides. Panel B shows the novel peptides that were too short (less than 6 amino acids) for the design of degenerate probes or degenerate PCR primers;

Figures 13 to 20 relate to Example 3 hereinafter, and show various aspects of the mitogenic activity of factors of the invention;

Figures 21 to 28 (a, b and c) relate to Example 4 hereinafter, and are briefly described below:

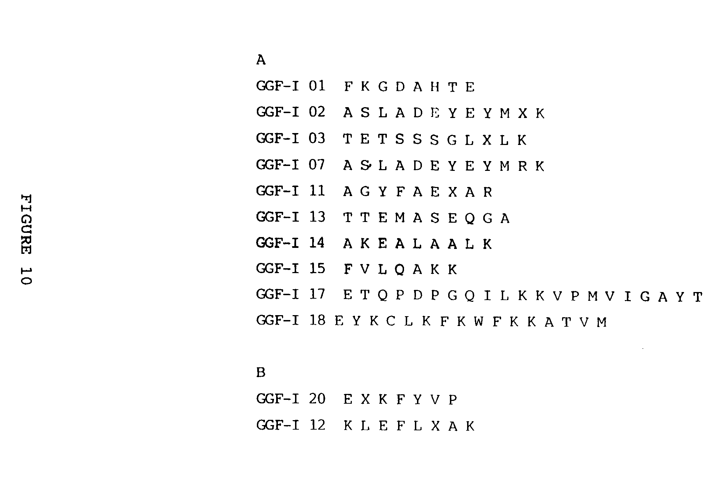

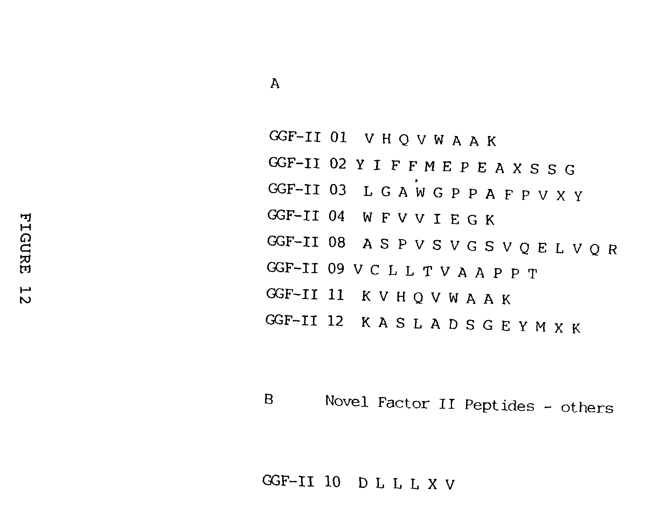

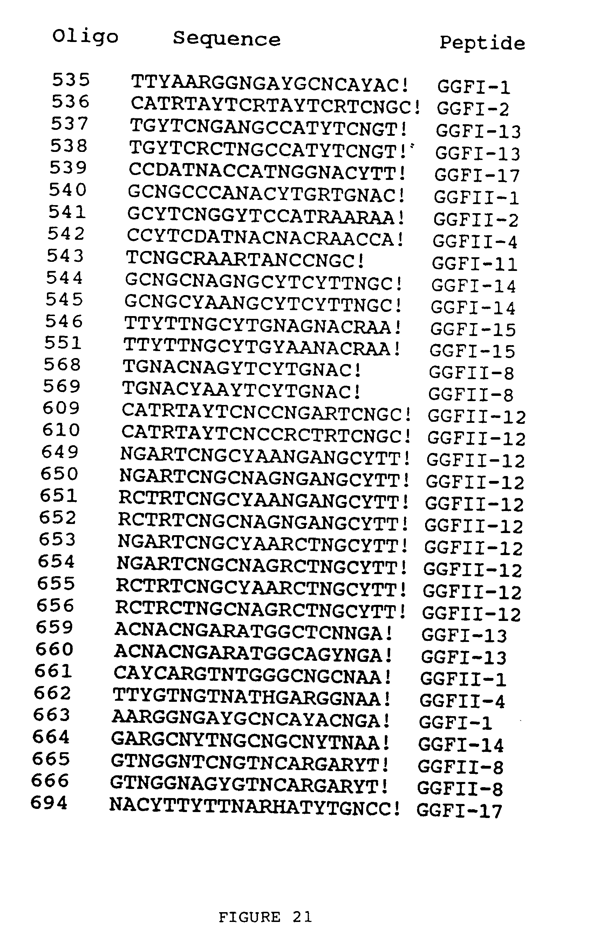

Figure 21 lists the degenerate oligonucleotide probes that were designed from the novel peptide sequences listed in Figure 10, Panel A and Figure 12, Panel A;

Figure 22 shows a stretch of the putative bovine GGF-II gene sequence from the recombinant bovine genomic phage GGF2BG1, which contains the binding site of degenerate oliognucleotide probes 609 and 650 (see Figure 21).
Shown are the coding strand of the DNA sequence and the deduced amino acid sequence in the third reading frame. The sequence of peptide 12 from factor 2 (bold) is part of a 66 amino acid open reading frame (nucleotides 75-272);

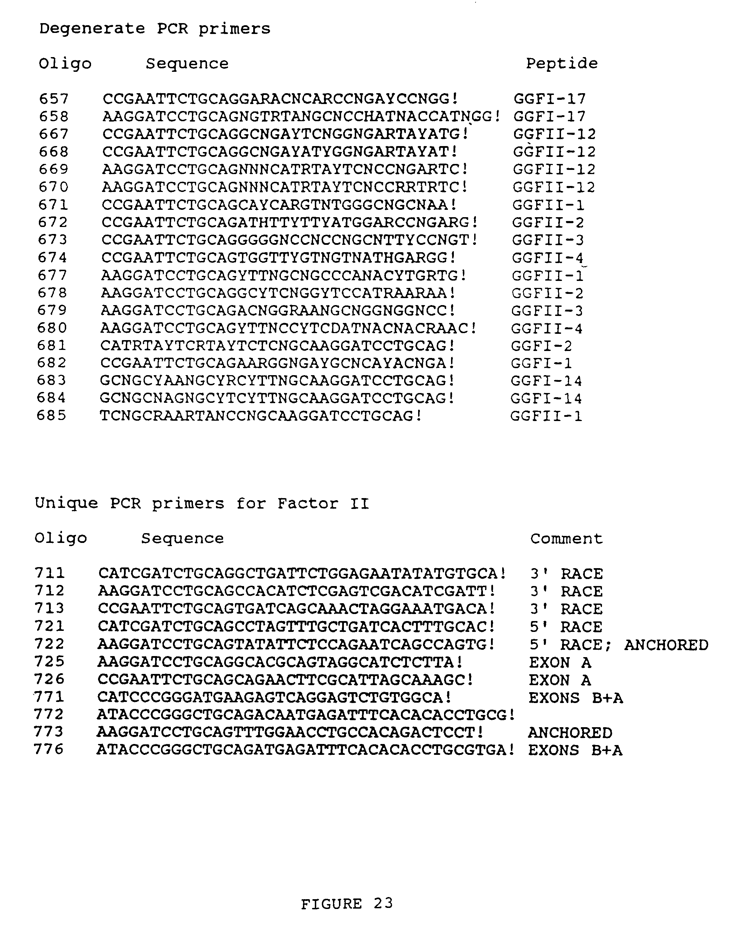

Figure 23 lists the degenerate PCR primers (Panel A) and unique PCR primers (Panel B) used in experiments to isolate segments of the bovine GGF-II coding sequences present in RNA from posterior pituitary;

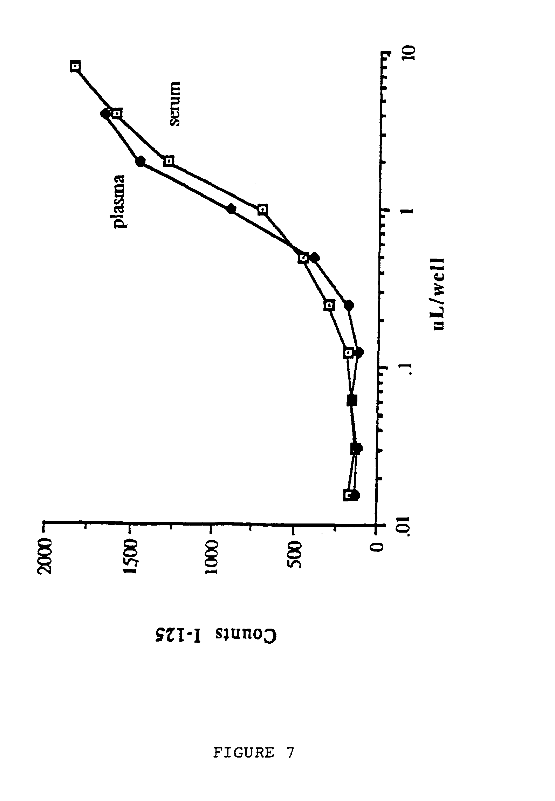

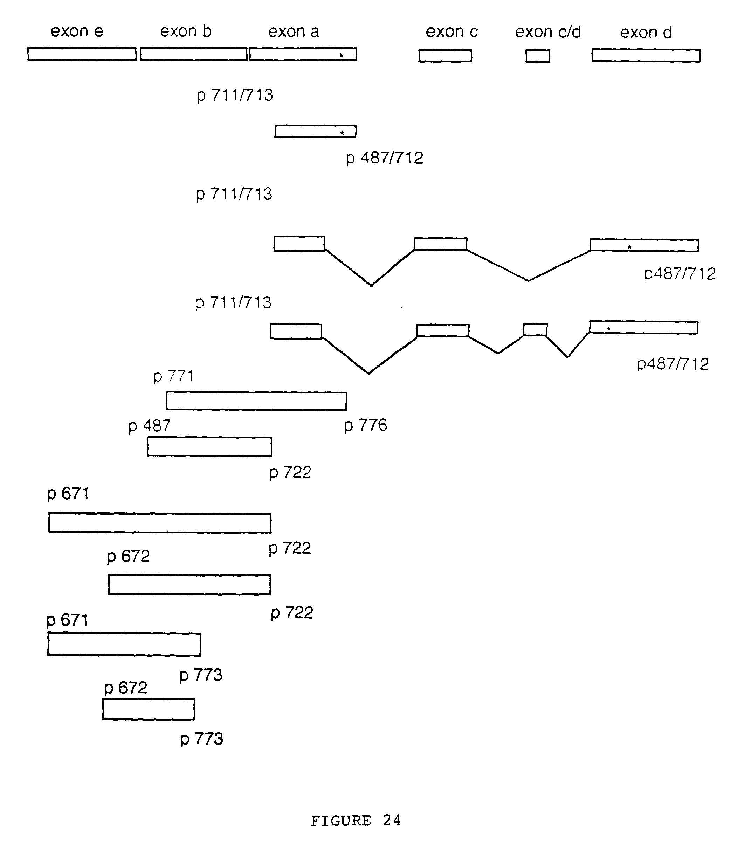

Figure 24 summarizes the nine distinct contiguous bovine GGF-II cDNA structures and sequences that were obtained in PCR amplification experiments using the list of primers in Figure 7, Panels A and B, on RNA from posterior pituitary. The top line of the Figure shows a schematic of the exon sequences which contribute to the cDNA structures that were characterized;

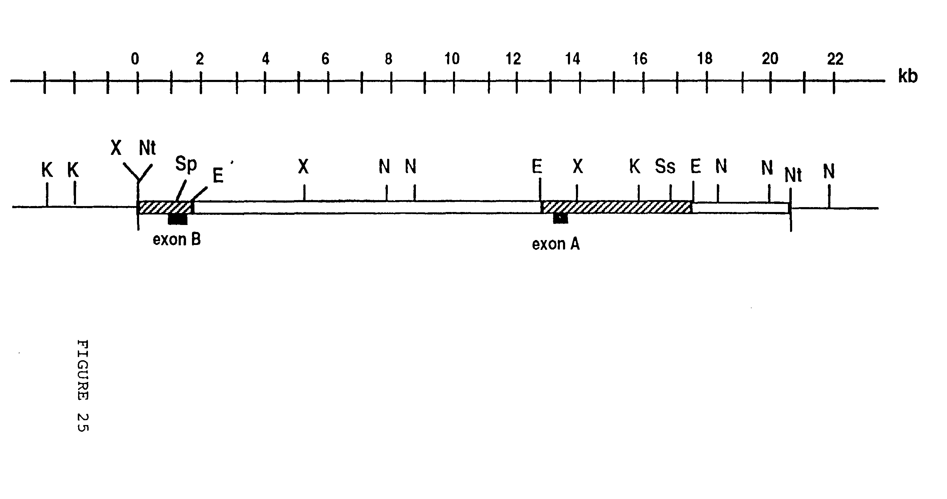

Figure 25 is a physical map of bovine recombinant phage of GGF2BG1. The bovine fragment is roughly 20 kb in length and contains two exons (bold) of the bovine GGF-II gene. Restriction sites for the enzymes Xbal, Spe I, Ndel, EcoRI, Kpnl, and SstI have been placed on this physical map. Shaded portions correspond to fragments which were subcloned for sequencing;

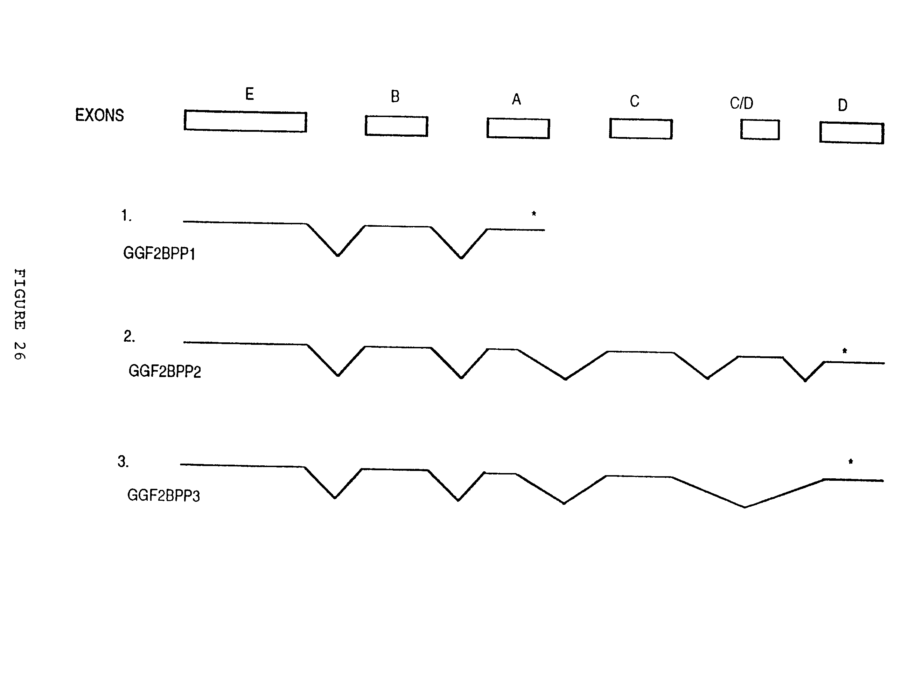

Figure 26 shows schematically the structure of three alternative gene products of the putative bovine GGF-II gene. Exons are listed A through E in the order of their discovery. The alternative splicing patterns 1, 2 and 3 generate three overlapping deduced protein structures (GGF2BPP1, 2, and 3), which are displayed in the various Figures 28;

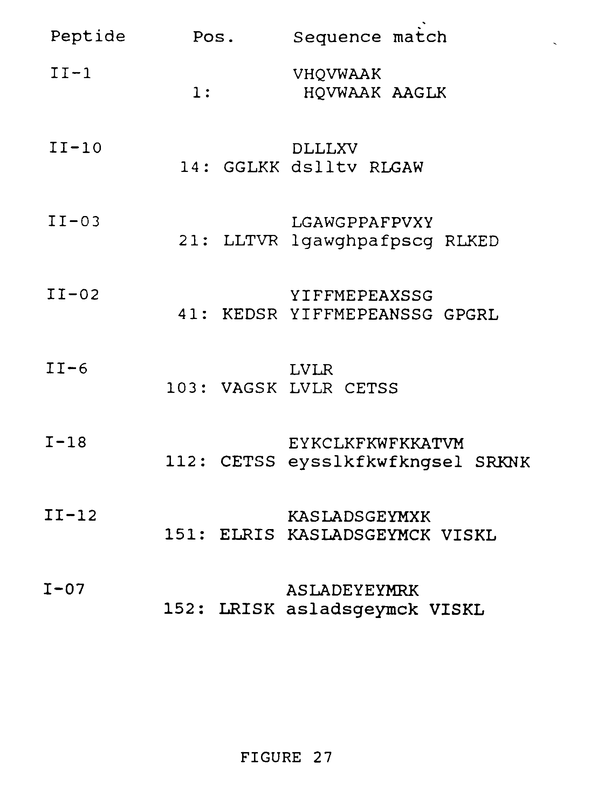

Figure 27 compares the GGF-I and GGF-II sequences identified in the deduced protein sequences shown in Figures 28a, 28b and 28c with the novel peptide sequences listed in Figures 10 and 12. The Figure shows that six of the nine novel GGF-II peptide sequences are accounted for in these deduced protein sequences. Two peptide sequences similar to GGF-I sequences are also found;

Figure 28a shows the coding strand DNA sequence and deduced amino acid sequence of the cDNA obtained from splicing pattern number 1 shown in Figure 26. This partial cDNA of the putative bovine GGF-II gene encodes a protein of 207 amino acids in length. Peptides shown in bold were those identified from the lists presented in Figures 10 and 12. Potential glycosylation sites are underlined (along with polyadenylation signal AATAAA);

Figure 28b shows the coding strand DNA sequence and deduced amino acid sequence of the cDNA obtained from splicing pattern number 2 shown in Figure 26. This partial cDNA of the putative bovine GGF-II gene encodes a protein of 264 amino acids in length. Peptides shown in bold were those identified from the lists presented in Figures 10 and 12. Potential glycosylation sites are underlined (along with polyadenylation signal AATAAA);

Figure 28c shows the coding strand DNA sequence and deduced amino acid sequence of the cDNA obtained from splicing pattern number 3 shown in Figure 26. This partial cDNA of the putative bovine GGF-II gene encodes a protein of 258 amino acids in length. Peptides shown in bold were those identified from the lists presented in Figures 10 and 12. Potential glycosylation sites are underlined (along with polyadenylation signal AATAAA); and

The DNA sequences shown in Figures 28a, 28b and 28c are themselves further aspects of this invention; and the invention further includes polypeptides encoded by said sequences;

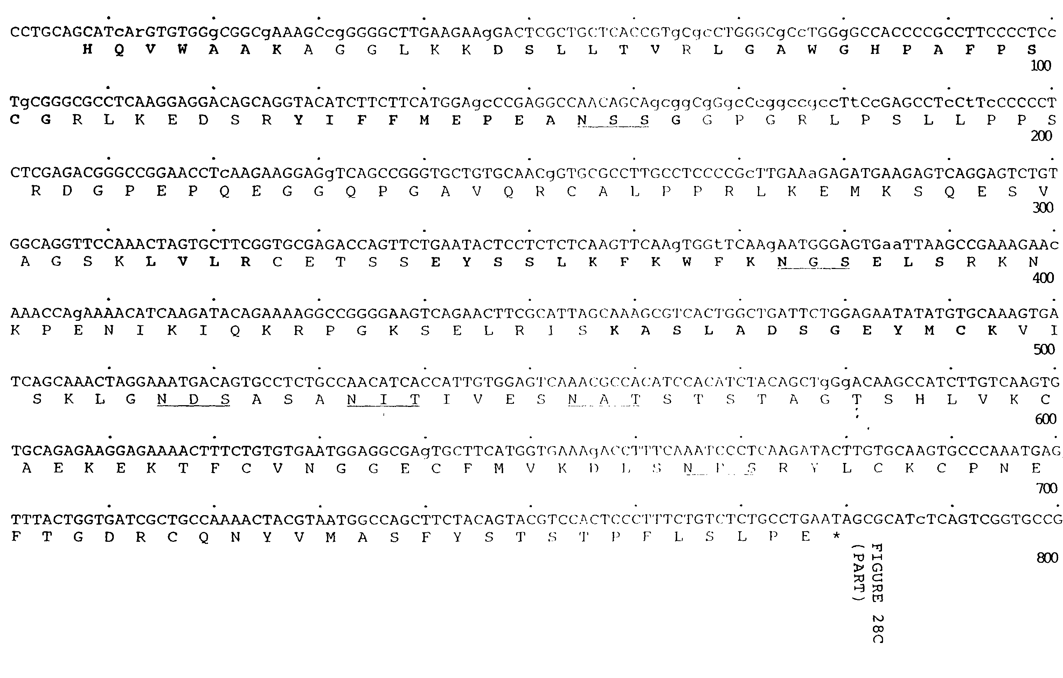

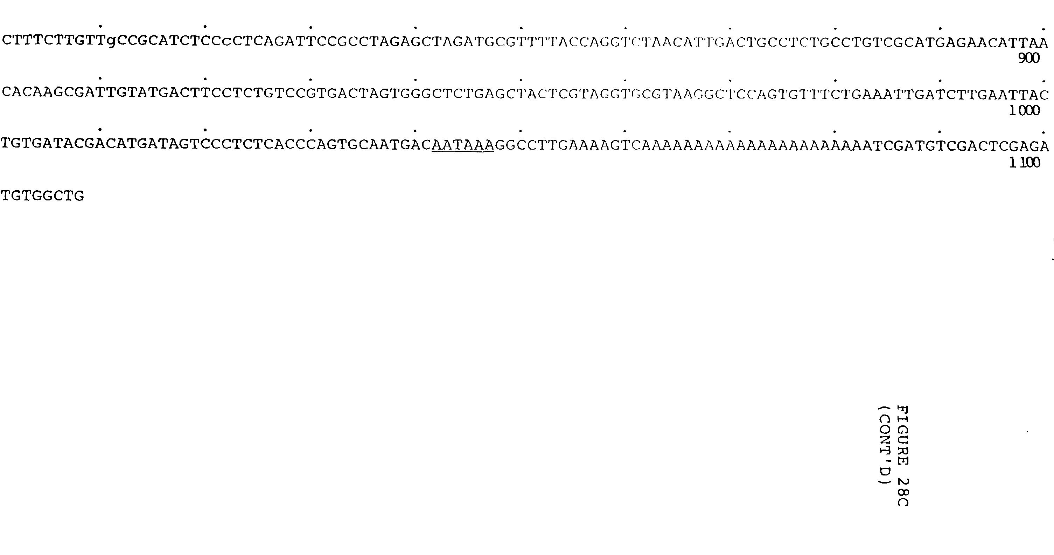

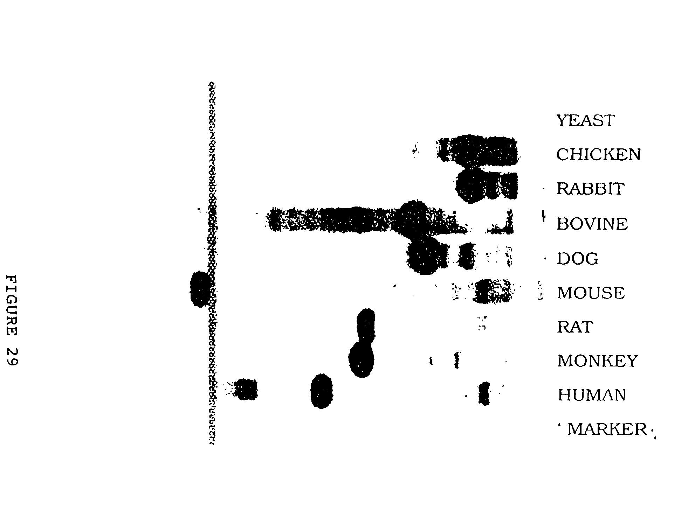

Figure 29 relates to Example 6 hereinafter, and shows an autoradiogram of a cross hybridization analysis of putative bovine GGF-II gene sequences to a variety of mammalian DNAs on a southern blot. The filter contains lanes of Eco RI-digested DNA (5 micrograms per lane) from the species listed in the Figure. The probe detects a single strong band in each DNA sample, including a four kb fragment in the bovine DNA as anticipated by the physical map in Figure 25. Bands of relatively minor intensity are observed as well, which could represent related DNA sequences. The strong hybridizing band from each of the other mammalian DNA samples presumably represents the GGF-II homologue of those species.

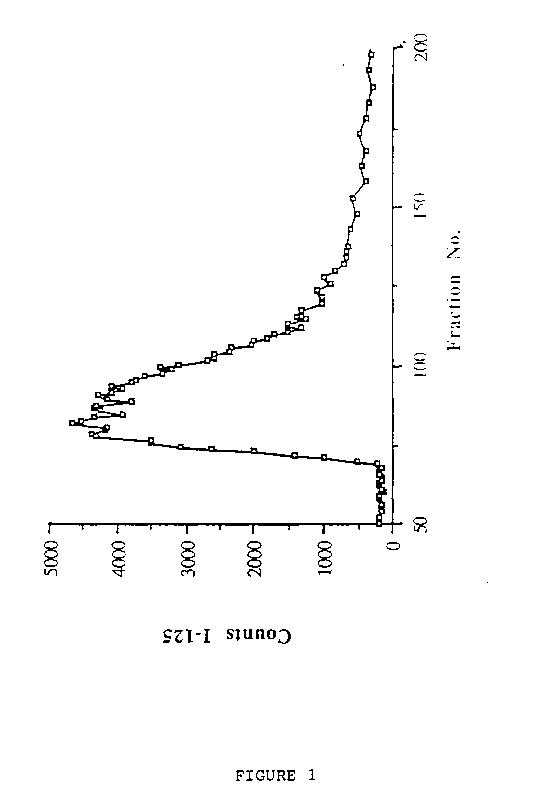

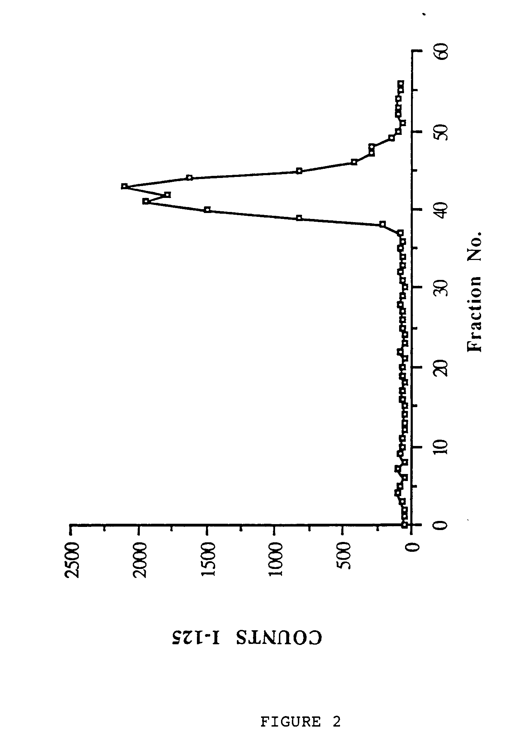

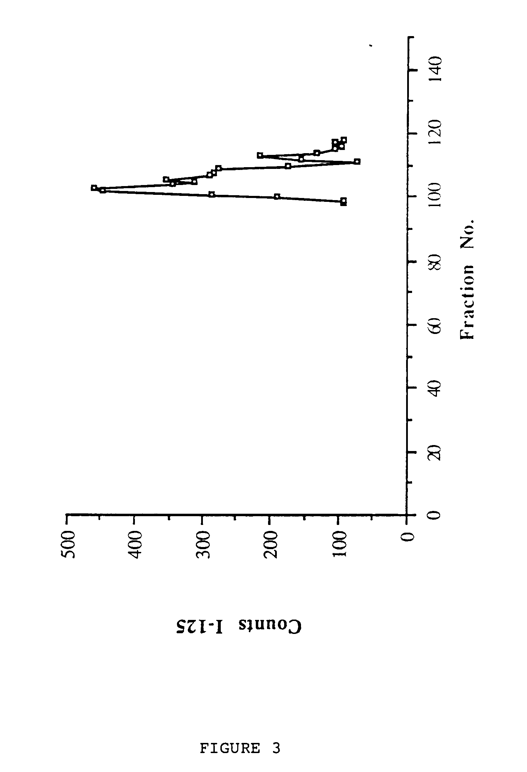

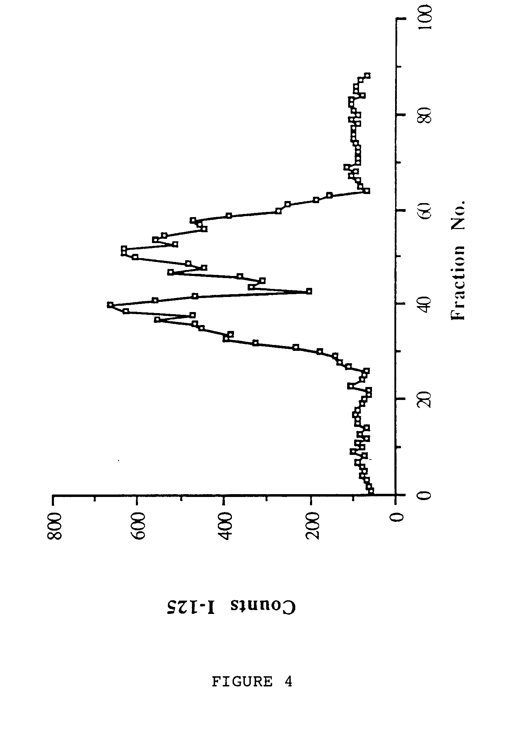

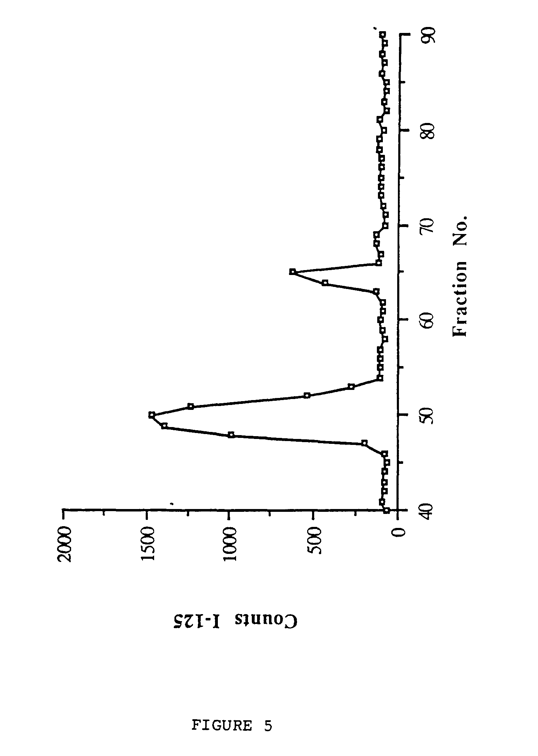

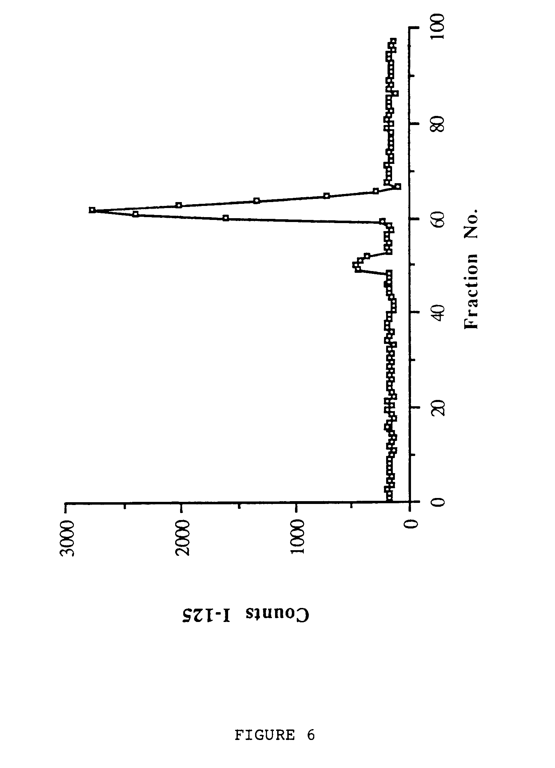

In Example 1 hereinafter, unless otherwise indicated, all operations were conducted at 4°C, and, with reference to Figures 1 to 6, activity at each stage was determined using the Brockes (Meth. Enz., supra) techniques with the following modifications. Thus, in preparing Schwann cells, 5μM forskolin was added in addition to DMEM (Dulbecco's modified Eagle's medium), FCS and GGF. Cells used in the assay were fibroblast-free Schwann cells at passage number less than 10, and these cells were removed from flasks with trypsin and plated into flat-bottomed 96-well plates at 3.3 thousand cells per microwell. [125I]IUdR was added for the final 24 hours after the test solution addition. The background (unstimulated) incorporation to each assay was less than 100 cpm, and maximal incorporation was 20 to 200 fold over background depending on Schwann cell batch and passage number.

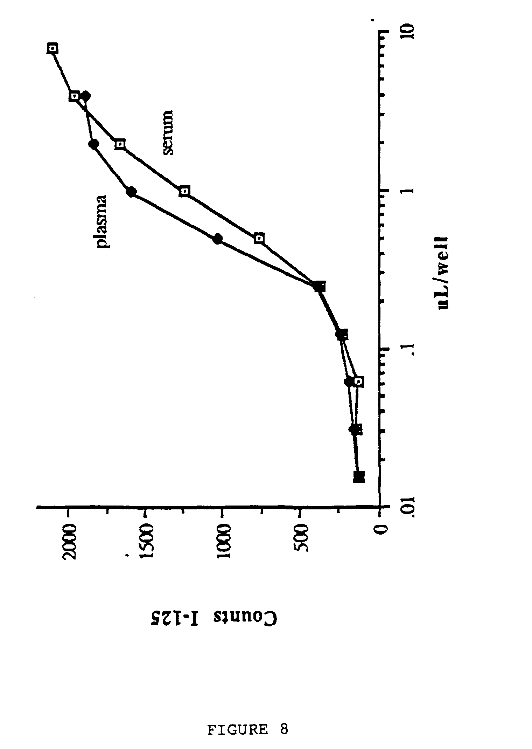

In the case of the GGF-I and GGF-II fractions from reversed-phase HPLC as described below in Example 1, two dose response curves were also produced for each factor, using exactly the above method for one of the curves for each factor, and the above method modified in the assay procedure only by substituting foetal calf plasma for foetal calf serum to obtain the other curve for each factor. The results are in Figures 7 and 8.

EXAMPLE 1

(A) Preparation of Factor-CM Fraction

4,000 frozen whole bovine pituitaries (c.a. 12 kg) were thawed overnight, washed briefly with water and then homogenised in an equal volume of 0.15 M ammonium sulphate in batches in a Waring Blender. The homogenate was taken to pH 4.5 with 1.0 M HCl and centrifuged at 4,900 g for 80 minutes. Any fatty material in the supernatant was removed by passing it through glass wool. After taking the pH of the supernatant to 6.5 using 1.0 M NaOH, solid ammonium sulphate was added to give a 36% saturated solution. After several hours stirring, the suspension was centrifuged at 4,900 g for 80 minutes and the precipitate discarded. After filtration through glass wool, further solid ammonium sulphate was added to the supernatant to give a 75% saturated solution which was once again centrifuged at 4,900 g for 80 minutes after several hours stirring. The pellet was resuspended in c.a. 2 L of 0.1 M sodium phosphate pH 6.0 and dialysed 3 x 40 L of the same buffer. After confirming that the conductivity of the dialysate was below 20.0 mSiemens, it was loaded on to a Bioprocess column (120 x 113 mm, Pharmacia) packed with carboxymethyl cellulose (CM-52, Whatman) at a flow rate of 2 ml.min$^{-1}$. The column was washed with 2 volumes of 0.1 M sodium phosphate pH 6.0, followed by 2 volumes of 50 mM NaCl, and finally 2 volumes of 0.2 M NaCl both in the same buffer. During the final step, 10 mL (5 minute) fractions were collected. Fractions 73 to 118 inclusive were pooled, dialysed against the 10 volumes of 10 mM sodium phosphate pH 6.0 twice and clarified by centrifugation at 100,000 g for 60 minutes.

(B) Hydroxylapatite HPLC

Hydroxylapatite HPLC is not a technique hitherto used in isolating glial growth factors, but proved particularly efficacious in this invention.

The material obtained from the above CM-cellulose chromatography was filtered through a 0.22 um filter (Nalgene), loaded at room temperature on to a high performance hydroxylapatite column (50 x 50 mm, Biorad) equipped with a guard column (15 x 25 mm, Biorad) and equilibrated with 10 mM potassium phosphate pH 6.0. Elution at room temperature was carried out at a flow rate of 2 mL.minute$^{-1}$ using the following programmed linear gradient:

| time (min) | %B | Solvent A | 10 mM potassium phosphate pH 6.0 |
|---|---|---|---|
| 0.0 | 0 | Solvent B | 1.0 M potassium phosphate pH 6.0 |
| 5.0 | 0 | | |
| 7.0 | 20 | | |
| 70.0 | 20 | | |
| 150.0 | 100 | | |
| 180.0 | 100 | | |
| 185.0 | 0 | | |

6.0 mL (3 minutes) fractions were collected during the gradient elution. Fractions 39-45 were pooled and dialysed against 10 volumes of 50 mM sodium phosphate pH 6.0.

(C) Mono S FPLC

Mono S FPLC enabled a more concentrated material to be prepared for subsequent gel filtration.

Any particulate material in the pooled material from the hydroxylapatite column was removed by a clarifying spin at 100,000 g for 60 minutes prior to loading on to a preparative HR10/10 Mono S cation exchange column (100 x 10 mm, Pharmacia) which was then re-equilibrated to 50 mM sodium phosphate pH 6.0 at room temperature with a flow rate of 1.0 mL.minute$^{-1}$. Under these conditions, bound protein was eluted using the following programmed linear gradient:

| time (min) | %B | Solvent A | 50 mM potassium phosphate pH 6.0 |
|---|---|---|---|
| 0.0 | 0 | Solvent B | 1.2 M sodium chloride, 50 mM |
| 70.0 | 30 | | sodium phosphate pH 6.0 |
| 240.0 | 100 | | |
| 250.0 | 100 | | |
| 260.0 | 0 | | |

1 mL (1 minute) fractions were collected throughout this gradient programme. Fractions 99 to 115 inclusive were pooled.

(D) Gel Filtration FPLC

This step commenced the separation of the two factors of the invention prior to final purification, producing enriched fractions.

For the purposes of this step, a preparative Superose 12 FPLC column (510 x 20 mm, Pharmacia) was packed according to the manufacturers' instructions. In order to standardize this column, a theoretical plates measurement was made according to the manufacturers' instructions, giving a value of 9,700 theoretical plates.

The pool of Mono S eluted material was applied at room temperature in 2.5 mL aliquots to this column in 50 mM sodium phosphate, 0.75 NaCl pH 6.0 (previously passed through a C18 reversed phase column (Sep-pak, Millipore)) at a flow rate of 1.0 mL.minute$^{-1}$. 1 mL (0.5 minute) fractions were collected from 35 minutes after each sample was applied to the column. Fractions 27 to 41 (GGF-II) and 42 to 57 (GGF-I) inclusive from each run were pooled.

(E) Reversed-Phase HPLC

The GGF-I and GGF-II pools from the above Superose 12 runs were each divided into three equal aliquots. Each aliquot was loaded on to a C8 reversed-phase column (Aquapore RP-300 7 µ C8 220 x 4.6 mm, Applied Biosystems) protected by a guard cartridge (RP-8, 15 x 3.2 mm, Applied Biosystems) and equilibrated to 40°C at 0.5 mL. minute$^{-1}$. Protein was eluted under these conditions using the following programmed linear gradient:

| time (min) | %B | Solvent A | 0.1% trifluoroacetic acid (TFA) |
|---|---|---|---|
| 0 | 0 | Solvent B | 90% acetonitrile, 0.1% TFA |
| 60 | 66.6 | | |
| 62.0 | 100 | | |
| 72.0 | 100 | | |
| 75.0 | 0 | | |

200 µL (0.4 minute) fractions were collected in siliconised tubes (Multilube tubes, Bioquote) from 15.2 minutes after the beginning of the programmed gradient.

(F) SDS-Polyacrylamide Gel Electrophoresis

In this step, protein molecular weight standards, low range, catalogue no. 161-0304, from Bio-Rad Laboratories Limited, Watford, England were employed. The actual proteins used, and their molecular weight standards, have been listed hereinbefore.

Fractions 47 to 53 (GGF-I) and fractions 61 to 67 (GGF-II) inclusive from the reversed-phase runs were individually pooled. 7 µL of the pooled material was boiled in an equal volume of 0.0125 M Tris-Cl, 4% SDS, 20% glycerol, and 10% β-mercaptoethanol for GGF-I, for 5 minutes and loaded on to an 11% polyacrylamide Leammli gel with a 4% stacking gel and run at a constant voltage of 50 V for 16 hours. This gel was then fixed and stained using a silver staining kit (Amersham). Under these conditions, the factors are each seen as a somewhat diffuse band at relative molecular weights 30,000 to 36,000 Daltons (GGF-I) and 55,000 to 63,000 Daltons (GGF-II) as defined by molecular weight markers. From the gel staining, it is apparent that there are a small number of other protein species present at equivalent levels to the GGF-I and GGF-II species in the material pooled from the reversed-phase runs.

Stability in Trifluoroacetic Acid

Stability data were obtained for the present Factors in the presence of trifluoroacetic acid, as follows:-

GGF-I

Material from the reversed-phase HPLC, in the presence of 0.1% TFA and acetonitrile, was assayed within 12 hours of the completion of the column run and then after 10 weeks incubation at 4°C. Following incubation, the GGF-I had at least 50% of the activity of that material assayed directly off the column.

GGF-II

Material from the reversed-phase HPLC, in the presence of 0.1% TFA and acetonitrile, and stored at -20°C, was assayed after thawing and then after 4 days incubation at 4°C. Following incubation, the GGF-II had at least 50% of the activity of that material freshly thawed.

It will be appreciated that the trifluoroacetic acid concentration used in the above studies is that most commonly used for reversed-phase chromatography.

EXAMPLE 2

Amino acid sequence analysis studies were performed using highly purified bovine pituitary GGF-I and GGF-II. The conventional single letter code was used to describe the sequences. Peptides were obtained by lysyl endopeptidase and protease V8 digests, carried out on reduced and carboxymethylated samples, with the lysyl endopeptidase digest of GGF-II carried out on material eluted from the 55-65 kD region of a 11% SDS-PAGE (MW relative to the above-quoted markers).

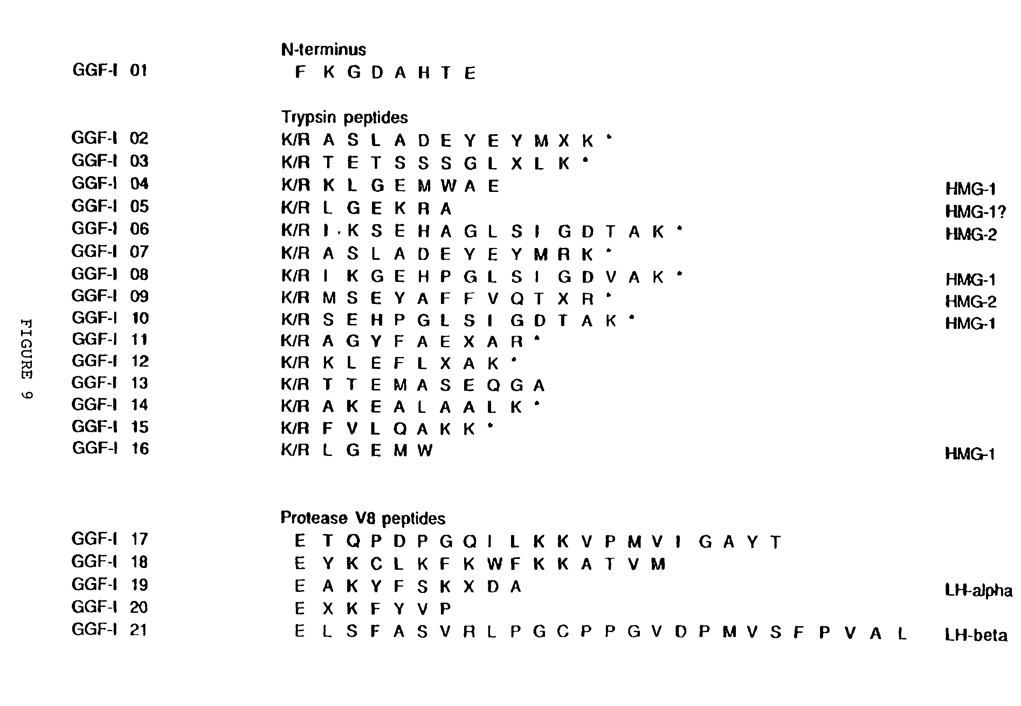

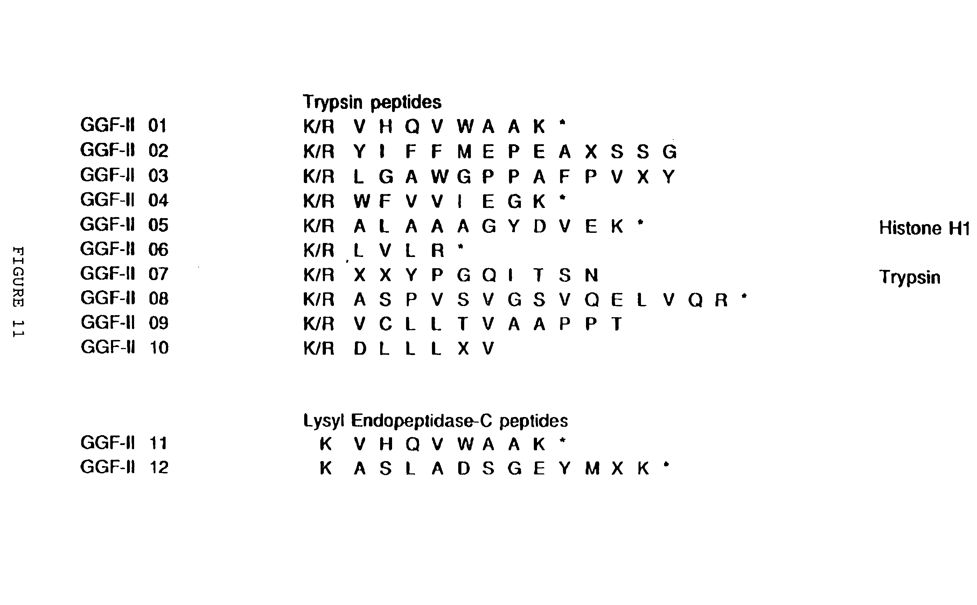

A total of 21 peptide sequences (see Figure 9) were obtained for GGF-I, of which 12 peptides (see Figure 10) are not present in current protein databases and therefore represent unique sequences. A total of 12 peptide sequences (see Figure 11) were obtained for GGF-II, of which 10 peptides (see Figure 12) are not present in current protein databases and therefore represent unique sequences (an exception is peptide GGF-II 06 which shows identical sequences in many proteins which are probably of no significance given the small number of residues). These novel sequences are extremely likely to correspond to portions of the true amino acid sequences of GGFs I and II.

Particular attention can be drawn to the sequences of GGF-I 07 and GGF-II 12, which are clearly highly related. The similarities indicate that the sequences of these peptides are almost certainly those of the assigned GGF species, and are most unlikely to be derived from contaminant proteins.

In addition, in peptide GGF-II 02, the sequence X S S is consistent with the presence of an N linked carbohydrate moiety on an asparagine at the position denoted by X.

In general, in Figures 9 and 11, X represents an unknown residue denoting a sequencing cycle where a single position could not be called with certainty either because there was more than one signal of equal size in the cycle or because no signal was present. As asterisk denotes those peptides where the last amino acid called corresponds to the last amino acid present in that peptide. In the remaining peptides, the signal strength after the last amino acid called was insufficient to continue sequence calling to the end of that peptide. The right hand column indicates the results of a computer database search using the GCG package FASTA and TFASTA programs to analyse the NBRF and EMBL sequence databases. The name of a protein in this column denotes identity of a portion of its sequence with the peptide amino acid sequence called allowing a maximum of two mismatches. A question mark denotes three mismatches allowed. The abbreviations used are as follows:

| HMG-1 | High Mobility Group protein-1 |
| HMG-2 | High Mobility Group protein-2 |
| LH-alpha | Luteinizing hormone alpha subunit |
| LH-beta | Luteinizing hormone beta subunit |

EXAMPLE 3

The mitogenic activity of a highly purified sample containing both GGFs I and II was studied using a quantative method, which allows a single microculture to be examined for DNA synthesis, cell morphology, cell number and expression of cell antigens. This technique has been modified from a method previously reported by Muir D et al., Analytical Biochemistry 185, 377-382, 1990. The main modifications are: 1) the use of uncoated microtiter plates, 2) the cell number per well, 3) the use of 5% Foetal Bovine Plasma (FBP) instead of 10% Foetal Calf Serum (FCS), and 4) the time of incubation in presence of mitogens and bromodeoxyuridine (BrdU), added simultaneously to the cultures. In addition the cell monolayer was not washed before fixation to avoid loss of cells, and the incubation time of monoclonal mouse anti-BrdU antibody and peroxidase-conjugated goat anti-mouse immunoglobulin (IgG) antibody were doubled to increase the sensitivity of the assay. The assay, optimized for rat sciatic nerve Schwann cells, has also been used for several cell lines, after appropriate modifications to the cell culture conditions.

Methods

On day 1, purified Schwann cells were plated onto uncoated 96 well plates in 5% FBP/Dulbecco's Modified Eagle Medium (DMEM) (5,000 cells/well). On day 2, GGFs or other test factors were added to the cultures, as well as BrdU at a final concentration of 10μM. After 48 hours (day 4) BrdU incorporation was terminated by aspirating the medium and cells were fixed with 200 μl/well of 70% ethanol for 20 min at room temperature. Next, the cells were washed with water and the DNA denatured by incubation with 100 μl 2N HC1 for 10 min at 37°C. Following aspiration, residual acid was neutralized by filling the wells with 0.1M borate buffer, pH 9.0, and the cells were washed with phosphate buffered saline (PBS). Cells were then treated with 50 μl of blocking buffer (PBS containing 0.1% Triton X 100 and 2% normal goat serum) for 15 min at 37°C. After aspiration, monoclonal mouse anti-BrdU antibody (Dako Corp., Santa Barbara, CA) (50 μl/well, 1.4 μg/ml diluted in blocking buffer) was added and incubated for two hours at 37°C. Unbound antibodies were removed by three washes in PBS containing 0.1% Triton X-100 and peroxidase-conjugated goat ant-mouse IgG antibody (Dako Corp., Santa Barbara, CA) (50 μl/well, 2 μg/ml diluted in blocking buffer) was added and incubated for one hour at 37°C. After three washes in PBS/Triton and a final rinse in PBS, wells received 100 μl/well of 50 μM phosphate/citrate buffer, pH 5.0, containing 0.05% of the soluble chromogen o-phenylenediamine (OPD) and 0.02% $H_2O_2$. The reaction was terminated after 5-20 min at room temperature, by pipetting 80 μl from each well to a clean plate containing 40 μl/well of 2N sulfuric acid. The absorbance was recorded at 490nm using a plate reader (Dynatech Labs.). The assay plates containing the cell monolayers were washed twice with PBS and immunocytochemically stained for BrdU-DNA by adding 100 μl/well of the substrate diaminobenzidine (DAB) and 0.02% $H_2O_2$ to generate an insoluble product. After 10-20 min the staining reaction was stopped by washing with water, and BrdU-positive nuclei observed and counted using an inverted microscope. Occasionally, negative nuclei were counterstained with 0.001% Toluidine blue and counted as before.

Cell Lines

Swiss 3T3 Fibroblasts

Cells, from Flow Labs, were maintained in DMEM supplemented with 10% FCS, penicillin and streptomycin, at 37°C in a humidified atmosphere of 10% CO2 in air. Cells were fed or subcultured every two days. For mitogenic assay, cells were plated at a density of 5,000 cells/well in complete medium and incubated for a week until cells were confluent and quiescent. The serum containing medium was removed and the cell monolayer washed twice with serum free-medium. 100 μl of serum free medium containing mitogens and 10μM BrdU were added to each well and incubated for 48 hours. Dose responses to GGFs and serum or PDGF (as a positive control) were performed.

BHK (Baby Hamster Kidney) 21 C13 Fibroblasts

Cells from European Collection of Animal Cell Cultures (ECACC), were maintained in Glasgow Modified Eagle Medium (GMEM) supplemented with 5% tryptose phosphate broth, 5% FCS, penicillin and streptomycin, at 37°C in a humidified atmosphere of 5% CO2 in air. Cells were fed or subcultured every two to three days. For mitogenic assay, cells were plated at a density of 2,000 cell/well in complete medium for 24 hours. The serum containing medium was then removed and after washing with serum free medium, replaced with 100 μl of 0.1% FCS containing GMEM or GMEM alone. GGFs and FCS or bFGF as positive controls were added, coincident with 10μM BrdU, and incubated for 48 hours. Cell cultures were then processed as described for Schwann cells.

C6 Rat Glioma Cell Line

Cells, obtained at passage 39, were maintained in DMEM containing 5% FCS, 5% Horse serum (HS), penicillin and streptomycin, at 37°C in a humidified atmosphere of 10% CO2 in air. Cells were fed or subcultured every three days. For mitogenic assay, cells were plated at a density of 2,000 cells/well in complete medium and incubated for 24 hours. Then medium was replaced with a mixture of 1:1 DMEM and F12 medium containing 0.1% FCS, after washing in serum free medium. Dose responses to GGFs, FCS and aFGF were then performed and cells were processed through the ELISA as previously described for the other cell types.

PC12 (Rat Adrenal Pheochromocytoma Cells)

Cells from ECACC, were maintained in RPMI 1640 supplemented with 10% HS, 5% FCS, penicillin and streptomycin, in collagen coated flasks, at 37°C in a humidified atmosphere of 5% CO2 in air. Cells were fed every three days by replacing 80% of the medium. For mitogenic assay, cells were plated at a density of 3,000 cells/well in complete medium, on collagen coated plates (50 μl/well collagen, Vitrogen Collagen Corp., diluted 1 : 50, 30 min at 37°C) and incubated for 24 hours. The medium was then placed with fresh RPMI either alone or containing 1 μM insulin or 1% FCS. Dose responses to FCS/HS (1 : 2) as positive control and to GGFs were performed as before. After 48 hours cells were fixed and the ELISA performed as previously described.

Results

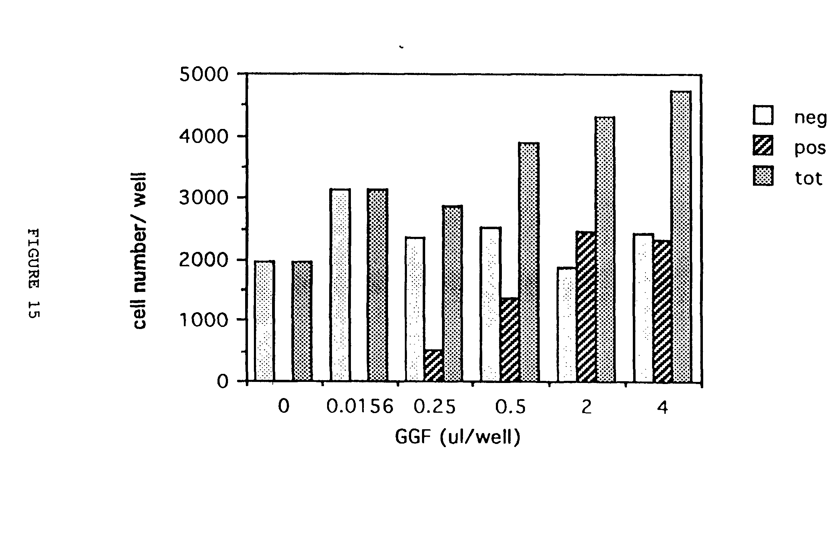

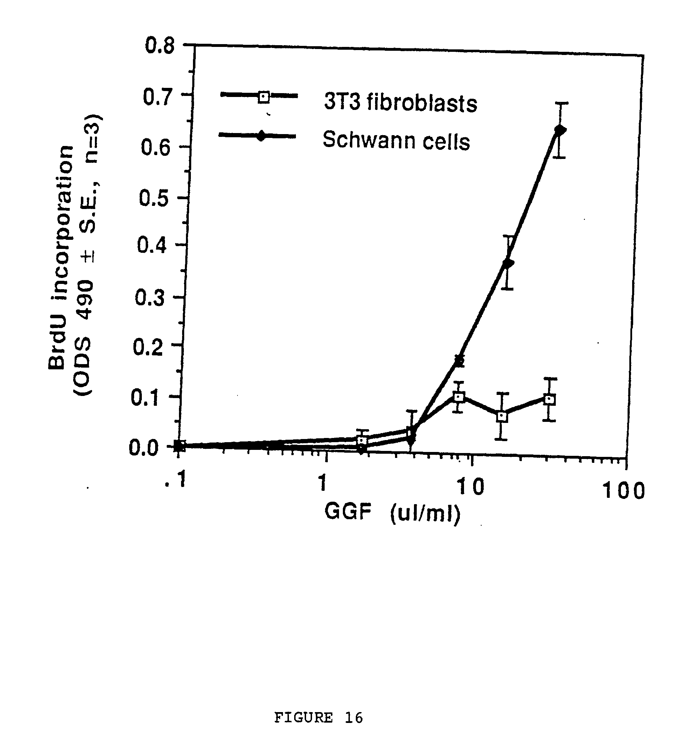

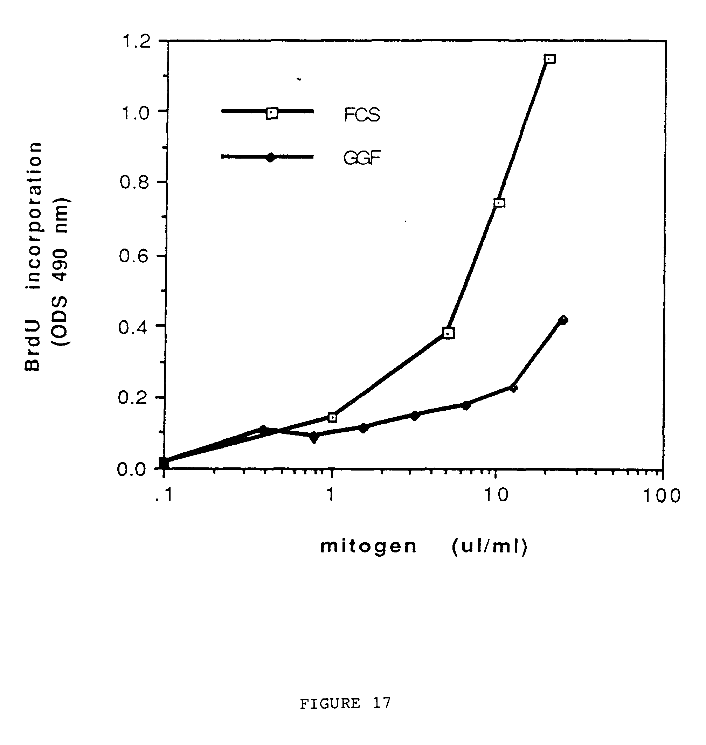

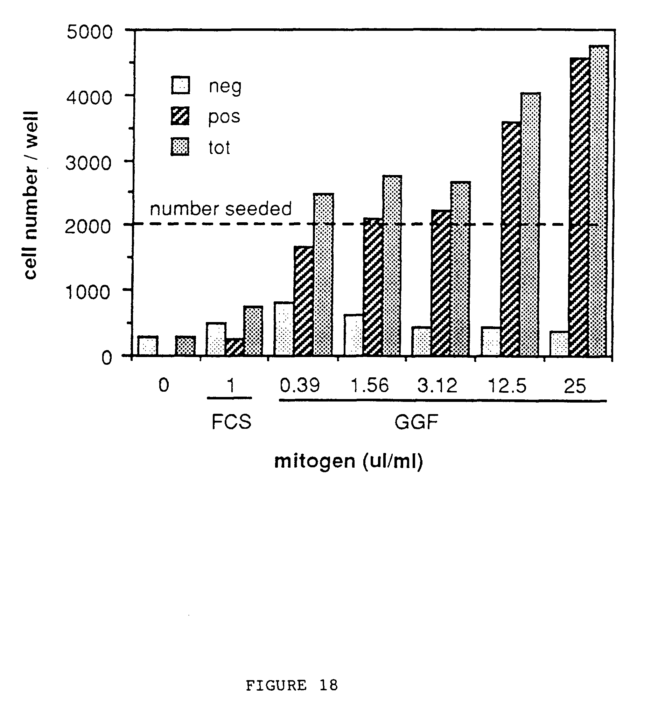

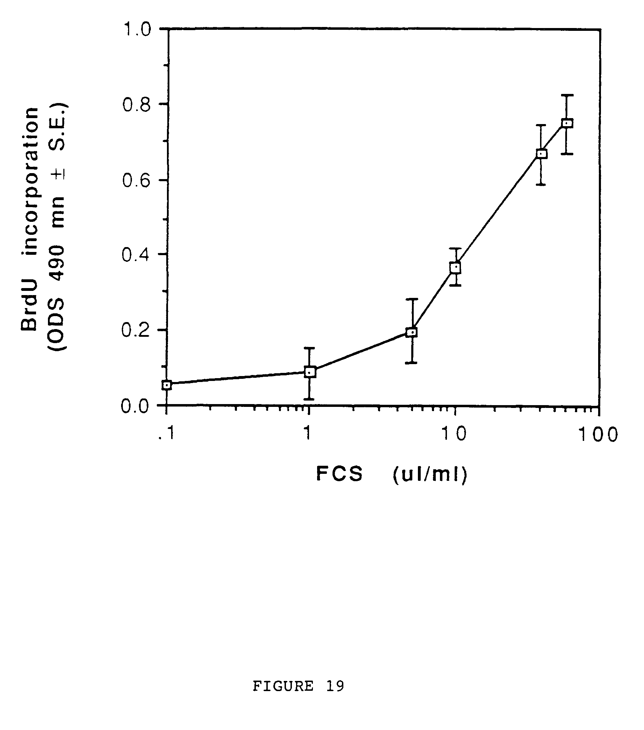

All the experiments presented in this Example were performed using a highly purified sample from a Superose 12 chromatography purification step (see Example 1, section D) containing a mixture of GGF-I and GGF-II (GGFs).
Firstly, the results obtained with the BrdU incorporation assay were compared with the classical mitogenic assay for Schwann cells based on [125]I-UdR incorporation into DNA of dividing cells, described by J.P.Brockes (Methods Enzymol. 147:217, 1987).

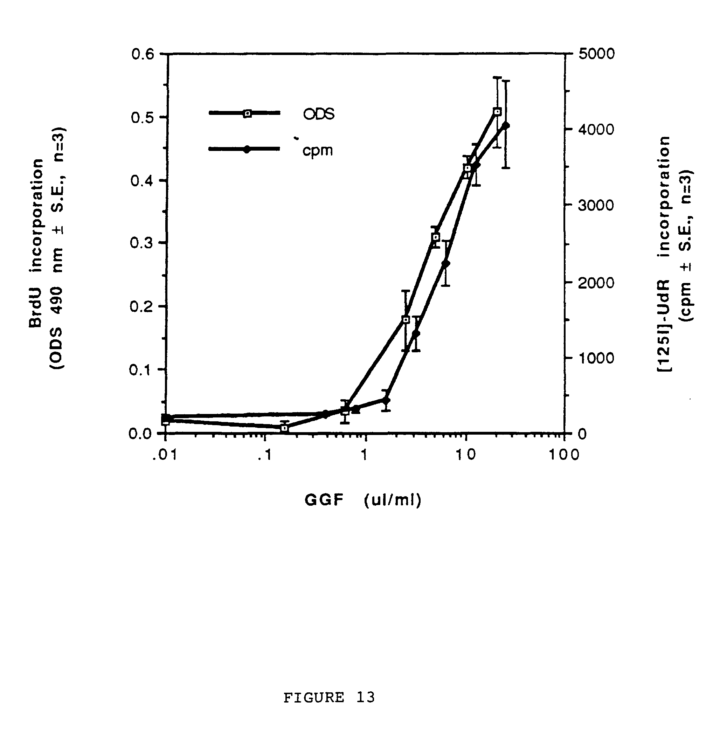

Figure 13 shows the comparison of data obtained with the two assays, performed in the same cell culture conditions (5,000 cells/well, in 5% FBP/DMEM, incubated in presence of GGFs for 48hrs). As clearly shown, the results are comparable, but BrdU incorporation assay appears to be slightly more sensitive, as suggested by the shift of the curve to the left of the graph, i.e. to lower concentrations of GGFs.
As described under the section "Methods", after the immunoreactive BrdU-DNA has been quantitated by reading the intensity of the soluble product of the OPD peroxidase reaction, the original assay plates containing cell monolayers can undergo the second reaction resulting in the insoluble DAB product, which stains the BrdU-positive nuclei. The microcultures can then be examined under an inverted microscope, and cell morphology and the numbers of BrdU-positive and negative nuclei can be observed.

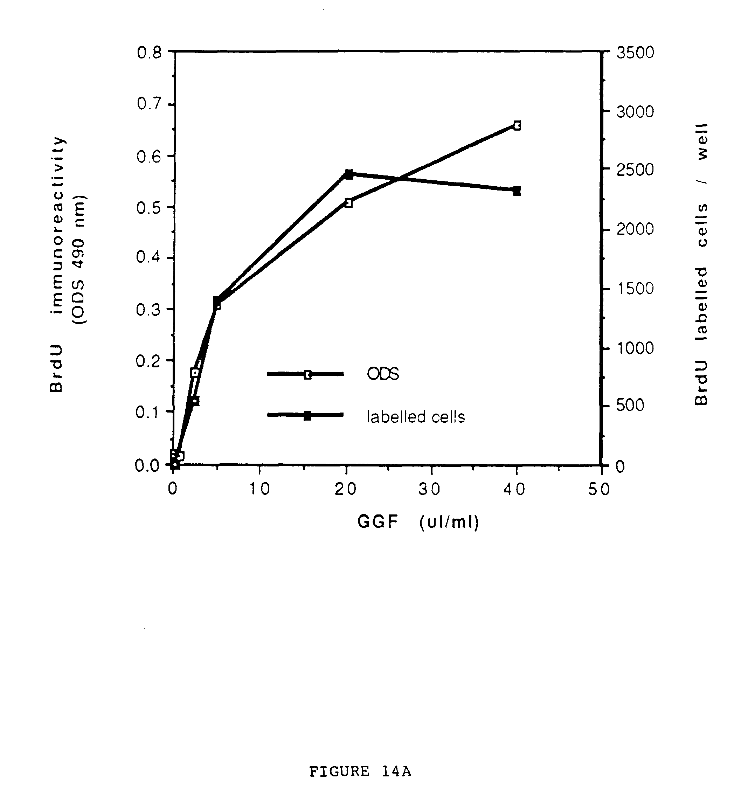

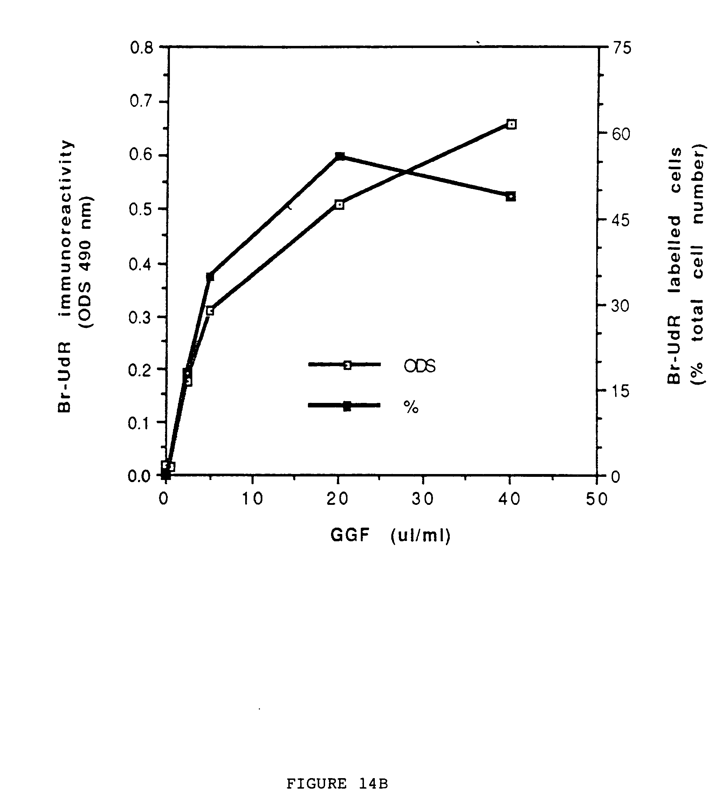

In Figure 14a and Figure 14b the BrdU-DNA immunoreactivity, evaluated by reading absorbance at 490 nm, is compared to the number of BrdU-positive nuclei and to the percentage of BrdU-positive nuclei on the total number of cells per well, counted in the same cultures. Standard deviations were less than 10%. The two evaluation methods show a very good correlation and the discrepancy between the values at the highest dose of GGFs can be explained by the different extent of DNA synthesis in cells detected as BrdU-positive.
The BrdU incorporation assay can therefore provide additional useful information about the biological activity of GGFs on Schwann cells when compared to the [125]I-UdR incorporation assay. For example, the data reported in Figure 15 show that GGFs can act on Schwann cells to induce DNA synthesis, but at lower doses to increase the number of negative cells present in the microculture after 48 hours.
The assay has then been used on several cell lines of different origin. In Figure 16 the mitogenic responses of Schwann cells and Swiss 3T3 fibroblasts to GGFs are compared; despite the weak response obtained in 3T3 fibroblasts, some clearly BrdU-positive nuclei were detected in these cultures. Control cultures were run in parallel in presence of several doses of FCS or human recombinant PDGF, showing that the cells could respond to appropriate stimuli (not shown).
The ability of fibroblasts to respond to GGFs was further investigated using the BHK 21 C13 cell line. These fibroblasts, derived from kidney, do not exhibit contact inhibition or reach a quiescent state when confluent. Therefore the experimental conditions were designed to have a very low background proliferation without comprising the cell viability. GGFs have a significant mitogenic activity on BHK21 C13 cells as shown by Figure 17 and Figure 18.
Figure 17 shows the BrdU incorporation into DNA by BHK 21 C13 cells stimulated by GGFs in the presence of 0.1% FCS. The good mitogenic response to FCS indicates that cell culture conditions were not limiting. In Figure 18 the mitogenic effect of GGFs is expressed as the number of BrdU-positive and BrdU-negative cells and as the total number of cells counted per well. Data are representative of two experiments run in duplicates; at least three fields per well were counted. As observed for Schwann cells in addition to a proliferative effect at low doses, GGFs also increase the numbers of non-responding cells surviving. The percentage of BrdU-positive cells is proportional to the increasing amounts of GGFs added to the cultures. The total number of cells after 48 hours in presence of higher doses of GGFs is at least doubled, confirming that GGFs induce DNA synthesis and proliferation in BHK21 C13 cells. Under the same conditions, cells maintained for 48 hours in the presence of 2% FCS showed an increase of about six fold (not shown).
C6 glioma cells have provided a useful model to study glial cell properties. The phenotype expressed seems to be dependent on the cell passage, the cells more closely resembling an astrocyte phenotype at an early stage, and an oligodendrocyte phenotype at later stages (beyond passage 70). C6 cells used in these experiments were from passage 39 to passage 52. C6 cells are a highly proliferating population, therefore the experimental conditions were optimized to have a very low background of BrdU incorporation. The presence of 0.1% serum was necessary to maintain cell viability without significantly affecting the mitogenic responses, as shown by the dose response to FCS (Figure 19).

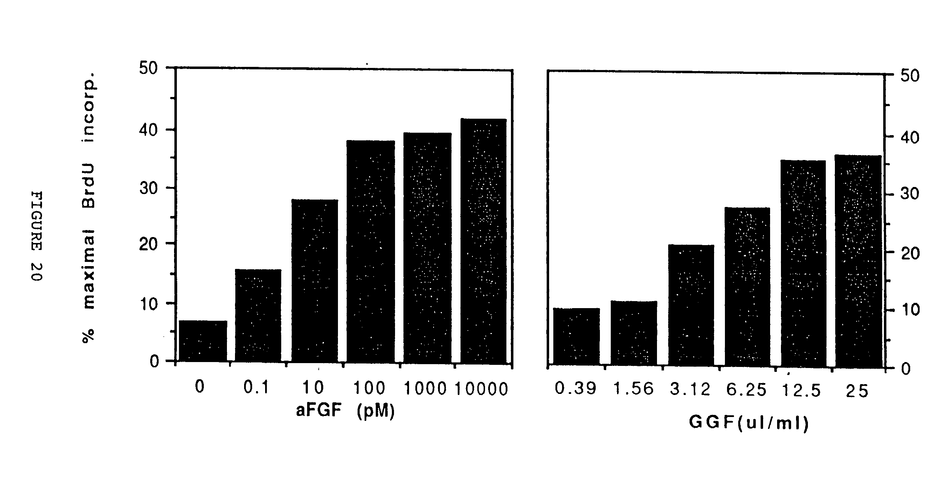

In Figure 20 the mitogenic responses to aFGF (Fibroblast growth factor) and GGFs are expressed as the percentages of maximal BrdU incorporation obtained in the presence of FCS (8%). Values are averages of two experiments, run in duplicates. The effect of GGFs was comparable to that of a pure preparation of aFGF. aFGF has been described as a specific growth factor for C6 cells (Lim R. et al., Cell Regulation 1:741-746, 1990) and for that reason it was used as a positive control. The direct counting of BrdU-positive and negative cells was not possible because of the high cell density in the microcultures.

In contrast to the cell lines so far reported, PC12 cells did not show any evident responsiveness to GGFs, when treated under culture conditions in which PC12 could respond to sera (mixture of FCS and HS as used routinely for cell maintenance). Nevertheless the number of cells plated per well seems to affect the behaviour of PC12 cells, and therefore further experiments are required.

EXAMPLE 4

Isolation and cloning of the GGF-II nucleotide sequence, using peptide sequence information and library screening, was performed as set out below. It will be appreciated that the peptides of Figures 9 and 10 can be used as the starting point for isolation and cloning of GGF-I sequences by following the techniques described herein. Indeed, Figure 21 shows possible degenerate oligonucleotide probes for this purpose, and Figure 23 lists possible PCR primers. DNA sequence and polypeptide sequence should be obtainable by this means as with GGF-II, and also DNA constructs and expression vectors incorporating such DNA sequence, host cells genetically altered by incorporating such constructs/vectors, and protein obtainable by cultivating such host cells. The invention envisages such subject matter.

1. Design and Synthesis of Oligonucleotide Probes and Primers

Degenerate DNA oligomer probes were designed by backtranslating the amino acid sequences (derived from the peptides generated from purified GGF protein) into nucleotide sequences. Oligomers represented either the coding strand or the non-coding strand of the DNA sequence. When serine, arginine or leucine were included in the oligomer design, then two separate syntheses were prepared to avoid ambiguities. For example, serine was encoded by either TCN or AGY as in 537 and 538 or 609 and 610. Similar codon splitting was done for arginine or leucine (e.g. 544, 545). DNA oligomers were synthesized on a Biosearch 8750 4-column DNA synthesizer using β-cyanoethyl chemistry operated at 0.2 micromole scale synthesis. Oligomers were cleaved off the column (500 angstrom CpG resins) and deprotected in concentrated ammonium hydroxide for 6-24 hours at 55-60°C.
Deprotected oligomers were dried under vacuum (Speedvac) and purified by electrophoresis in gels of 15% acrylamide (20 mono : 1 bis), 50 mM Tris-borate-EDTA buffer containing 7M urea. Full length oligomers were detected in the gels by UV shadowing, then the bands were excised and DNA oligomers eluted into 1.5 mls $H_2O$ for 4-16 hours with shaking. The eluate was dried, redissolved in 0.1 ml $H_2O$ and absorbance measurements were taken at 260nm. Concentrations were determined according to the following formula:

$$(A_{260} \text{ x units/ml}) (60.6/\text{length} = x \ \mu M)$$

All oligomers were adjusted to 50 $\mu M$ concentration by addition of $H_2O$.
Degenerate probes designed as above are shown in Figure 21.
PCR primers were prepared by essentially the same procedures that were used for probes with the following modifications. Linkers of thirteen nucleotides contained restriction sites were included at the 5' ends of the degenerate oligomers for use in cloning into vectors.
DNA synthesis was performed at 1 micromole scale using 1,000 angstrom CpG resins and inosine was used at positions where all four nucleotides were incorporated normally into degenerate probes. Purifications of PCR primers included an ethanol precipitation following the gel electrophoresis purification.

2. Library Construction and Screening

A bovine genomic DNA library was purchased from Stratagene (Catalogue Number: 945701). The library contained 2 x 10⁶ 15-20kb Sau3A1 partial bovine DNA fragments cloned into the vector lambda DashII. A bovine total brain cDNA library was purchased from Clonetech (Catalogue Number: BL 10139). Complementary DNA libraries were constructed (In Vitrogen; Stratagene) from mRNA prepared from bovine total brain, from bovine pituitary and from bovine posterior pituitary. In Vitrogen prepared two cDNA libraries: one library was in the vector lambda gt10, the other in vector pCDNAI (a plasmid library). The Stratagene libraries were prepared in the vector lambda unizap. Collectively, the cDNA libraries contained 14 million primary recombinant phage.
The bovine genomic library was plated on E. coli K12 host strain LE392 on 23 x 23 cm plates (Nunc) at 150,000 to 200,000 phage plaques per plate. Each plate represented approximately one bovine genome equivalent. Following an overnight incubation at 37°C, the plates were chilled and replicate filters were prepared according to procedures of Grunstein and Hogness [PNAS (USA (1975) 72:3961)]. Four plaque lifts were prepared from each plate onto uncharged nylon membranes (Pall Biodyne A or MSI Nitropure). The DNA was immobilized onto the membranes by cross-linking under UV light for 5 minutes or, by baking at 80° under vacuum for two hours.

DNA probes were labelled using T4 polynucleotide kinase (New England Biolabs) with gamma 32P ATP (New England Nuclear; 6500 Ci/mmol) according to the specifications of the suppliers. Briefly, 50 pmols of degenerate DNA oligomer were incubated in the presence of 600 μCi γ-32P-ATP and 5 units T4 polynucleotide kinase for 30 minutes at 37°C. Reactions were terminated, gel electrophoresis loading buffer was added and then radiolabelled probes were purified by electrophoresis. 32P labelled probes were excised from gel slices and eluted into water. Alternatively, DNA probes were labelled via PCR amplification by incorporation of α-32P-dATP or α-32P dCTP according to the protocol of Schowalter and Sommer, Anal. Biochem 177:90-94(1989). Probes labelled in PCR reactions were purified by desalting on Sephadex G-150 columns.

Prehybridization and hybridization were performed in GMC buffer (0.52M NaPi, 7% SDS, 1% BSA, 1.5mM EDTA, 0.1MNaCl, 10mg/ml tRNA). Washing was performed in buffer A oligowash (160ml 1M Na$_2$HPO$_4$, 200 ml 20% SDS, 8.0 ml 0.5m EDTA, 100 ml 5M NaCl, 3632 ml H$_2$O). Typically, 20 filters (400 sq. centimetres each) representing replicate copies of ten bovine genome equivalents were incubated in 200 ml hybridization solution with 100 pmols of degenerate oligonucleotide probe (128-512 fold degenerate). Hybridization was allowed to occur overnight at 5°C below the minimum melting temperature calculated for the degenerate probe. The calculation of minimum melting temperature assumes 2°C for an AT pair and 4°C for a GC pair.

Filters were washed in repeated changes of oligowash at the hybridization temperatures four to five hours and finally, in 3.2M tetramethylammonium chloride, 1% SDS twice for 30 min at a temperature dependent on the DNA probe length. For 20mers, the final wash temperature was 60°C. Filters were mounted, then exposed to X-ray film (Kodak XAR5) using intensifying screens (Dupont Cronex Lightening Plus). Usually, a three to five day film exposure at minus 80°C was sufficient to detect duplicate signals in these library screens.

Following analysis of the results, filters could be stripped and reprobed. Filters were stripped by incubating through two successive cycles of fifteen minutes in a microwave oven at full power in a solution of 1% SDS containing 10mM EDTA pH8. Filters were taken through at least three to four cycles of stripping and reprobing with various probes.

3. Recombinant Phage Isolation, Growth and DNA Preparation

These procedures followed standard protocol as described in Recombinant DNA (Maniatis et al 2:60-2:81).

4. Analysis of Isolated Clones Using DNA Digestion and Southern Blots

Recombinant Phage DNA samples (2 micrograms) were digested according to conditions recommended by the restriction endonuclease supplier (New England Biolabs). Following a four hour incubation at 37°C, the reactions products were precipitated in the presence of 0.1M sodium acetate and three volumes of ethanol. Precipitated DNA was collected by centrifugation, rinsed in 75% ethanol and dried. All resuspended samples were loaded onto agarose gels (typically 1% in TAE buffer; 0.04M Tris acetate, 0.002M EDTA). Gel runs were at 1 volt per centimetre from 4 to 20 hours. Markers included lambda Hind III DNA fragments and/or φX174HaeIII DNA fragments (New England Biolabs). The gels were stained with 0.5 micrograms/ml of ethidium bromide and photographed. For southern blotting, DNA was first depurinated in the gel by treatment with 0.125 N HC1, denatured in 0.5 N NaOH and transferred in 20x SSC (3M sodium chloride, 0.03 M sodium citrate) to uncharged nylon membranes. Blotting was done for 6 hours up to 24 hours, then the filters were neutralized in .1M Tris NaOH pH 7.5, 0.15 M sodium chloride, then rinsed briefly in 50 mM Tris-borate EDTA. For cross-linking, the filters were wrapped first in transparent plastic wrap, then the DNA side exposed for five minutes to an ultraviolet light. Hybridization and washing was performed as described for library screening (see section 2 of this Example). For hybridization analysis to determine whether similar genes exist in other species slight modifications were made. The DNA filter was purchased from Clonetech (Catalogue Number 7753-1) and contains 5 micrograms of EcoRI digested DNA from various species per lane. The probe was labelled by PCR amplification reactions as described in section 2 above, and hybridizations were done in 80% buffer B(2g polyvinylpyrrolidine, 2g Ficoll-400, 2g bovine serum albumin, 50ml 1M Tris-HC1(pH 7.5) 58g NaCl, 1g sodium pyrophosphate, 10g sodium dodecyl sulfate, 950ml H$_2$O) containing 10% dextran sulfate. The probes were denatured by boiling for ten minutes then rapidly cooling in ice water. The probe was added to the hybridization buffer at 10$^6$ dpm 32P per ml and incubated overnight at 60°C. The filters were washed at 60°C first in buffer B followed by 2X SSC, 0.1% SDS then in 1x SSC, 0.1% SDS. For high stringency, experiments, final washes were done in 0.1 X SSC, 1% SDS and the temperature raised to 65°C. Southern blot data were used to prepare a restriction map of the genomic clone and to indicate which subfragments hybridized to the GGF probes (candidates for subcloning).

5. Subcloning of Pieces of DNA Homologous to Hybridization Probes

DNA digests (e.g. 5 micrograms) were loaded onto 1% agarose gels then appropriate fragments excised from the gels following staining. The DNA was purified by adsorption onto glass beads followed by elution using the protocol described by the supplier (Bio 101). Recovered DNA fragments (100-200 ng) were ligated into linearized dephosphorylated vectors, e.g. pT3T7 (Ambion), which is a derivative of pUC18, using T4 ligase (New England Biolabs). This vector carries the E. coli β lactamase gene, hence, transformants can be selected on plates containing ampicillin. The vector also supplies β-galactosidase complementation to the host cell,therefore non-recombinants (blue) can be detected using isopropylthiogaloctoside and Bluogal (Bethesda Research Labs). A portion of the ligation reactions was used to transform E. coli K12 XL1 blue competent cells (Stratagene Catalogue Number: 200236) and then the transformants were selected on LB plates containing 50 micrograms per ml ampicillin. White colonies were selected and plasmid mini preps were prepared for DNA digestion and for DNA sequence analysis. Selected clones were retested to determine if their insert DNA hybridized with the GGF probes.

6. DNA Sequencing

Double stranded plasmid DNA templates were prepared from double stranded plasmids isolated from 5ml cultures according to standard protocols. Sequencing was by the dideoxy chain termination method using Sequenase 2.0 and a dideoxynucleotide sequencing kit (US Biochemical) according to the manufacturers protocol [a modification of Sanger et al. PNAS; USA 74:5463 (1977)]. Alternatively, sequencing was done in a DNA thermal cycler (Perkin Elmer, model 4800) using a cycle sequencing kit (New England Biolabs; Bethesda Research Laboratories) and was performed according to manufacturers instructions using a 5'-end labelled primer. Sequence primers were either those supplied with the sequencing kits or were synthesized according to sequence determined from the clones. Sequencing reactions were loaded on and resolved on 0.4mm thick sequencing gels of 6% polyacylamide. Gels were dried and exposed to X-Ray film. Typically, 35S was incorporated when standard sequencing kits were used and a 32P end labelled primer was used for cycle sequencing reactions. Sequences were read into a DNA sequence editor from the bottom of the gel to the top (5' direction to 3') and data were analyzed using programs supplied by Genetics Computer Group (GCG, University of Wisconsin).

7. RNA Preparation and PCR Amplification

Open reading frames detected in the genomic DNA and which contained sequence encoding GGF peptides were extended via PCR amplification of pituitary RNA. RNA was prepared from frozen bovine tissue (Pelfreeze) according to the guanidine neutral-CsCl chloride procedure [Chirgwin et. al. Biochemistry 18:5294(1979).] Polyadenylated RNA was selected by oligo-dT cellulose column chromatography [Aviv and Leder PNAS (USA) 69:1408 (1972)].
Specific DNA target sequences were amplified beginning with either total RNA or polyadenylated RNA samples that had been converted to cDNA using the Perkin Elmer PCR/RNA Kit Number: N808-0017. First strand reverse transcription reactions used 1 μg template RNA and either primers of oligo dT with restriction enzyme recognition site linkers attached or specific antisense primers determined from cloned sequences with restriction sites attached. To produce the second strand, the primers either were plus strand unique sequences as used in 3' RACE reactions [Frohman et. al., PNAS (USA)85:8998 (1988)] or were oligo dT primers with restriction sites attached if the second target site had been added by terminal transferase tailing first strand reaction products with dATP (e.g. 5' race reactions, Frohman et. al., ibid). Alternatively, as in anchored PCR reactions the second strand primers were degenerate, hence, representing particular peptide sequences.
The amplification profiles followed the following general scheme: 1) five minutes soak file at 95°C; 2) thermal cycle file of 1 minute, 95°C; 1 minute ramped down to an annealing temperature of 45°C, 50°C or 55°C; maintain the annealing temperature for one minute; ramp up to 72°C over one minute; extend at 72°C for one minute or for one minute plus a 10 second auto extension; 3) extension cycle at 72°C, five minutes, and; 4) soak file 4°C for infinite time. Thermal cycle files (#2) usually were run for 30 cycles. Sixteen μl of each 100 μl amplification reaction was analyzed by electrophoresis in 2% Nusiev 1% agarose gels run in TAE buffer at 4 volts per centimetre for three hours. The gels were stained, then blotted to uncharged nylon membranes which were probed with labelled DNA probes that were internal to the primers.
Specific sets of DNA amplification products could be identified in the blotting experiments and their positions used as a guide to purification and reamplification. When appropriate, the remaining portions of selected samples were loaded on to preparative gels, then following electrophoresis four to five slices of 0.5 mm thickness (bracketing the expected position of the specific product) were taken from the gel. The agarose was crushed, then soaked in 0.5 ml of electrophoresis buffer from 2-16 hours at 40°C. The crushed agarose was centrifuged for two minutes and the aqueous phase was transferred to fresh tubes. Reamplification was done on five microlitres (roughly 1% of the product) of the eluted material using the same sets of primers and the reaction profiles as in the original reactions. When the reamplification reactions were completed, samples were extracted with chloroform and transferred to fresh tubes. Concentrated restriction enzyme buffers and enzymes were added to the reactions in order to cleave at the restriction sites present in the linkers. The digested PCR products were purified by gel electrophoresis, then subcloned into vectors as described in the subcloning section above. DNA sequencing was done described as above.

8. DNA Sequence Analysis

DNA sequences were assembled using a fragment assembly program and the amino acid sequences deduced by the GCG programs GelAssemble, Map and Translate. The deduced protein sequences were searched using Word Search. Analysis was done on a VAX Station 3100 workstation operating under VMS 5.1. The database search was done on SwissProt release number 21 using GCG Version 7.0.

9. Results

As indicated, to identify the DNA sequence encoding bovine GGF-II degenerate oligonucleotide probes were designed from GGF-II peptide sequences. GGF-II 12, a peptide generated via lysyl endopeptidase digestion of a purified GGF-II preparation (see Figures 11 and 12) showed strong amino acid sequence homology with GGF-I 07, a tryptic peptide generated from a purified GGF-I preparation. GGF-II 12 was thus used to create ten degenerate oligonucleotide probes (see oligos 609, 610 and 649 to 656 in Figure 21). A duplicate set of filters were probed with two sets (set 1=609, 610; set 2=649-656) of probes encoding two overlapping portions of GGF-II 12. 46 hybridization signals were observed, however only one clone hybridized to both probe sets. The clone (designated GGF2BG1) was purified.
Southern blot analysis of DNA from the phage clone GGF2BG1 confirmed that both sets of probes hybridized with that bovine DNA sequence, and showed further that both probes reacted with the same set of DNA fragments within the clone. Based on those experiments a 4 kb Eco RI sub-fragment of the original clone was identified, subcloned and partially sequenced. Figure 22 shows the nucleotide sequence and the deduced amino acid sequence of the initial DNA sequence readings that included the hybridization sites of probes 609 and 650, and confirmed that a portion of this bovine genomic DNA encoded peptide 12 (KASLADSGEYM).
Further sequence analysis demonstrated that GGF-II 12 resided on a 66 amino acid open reading frame (see below) which has become the focal point for the isolation of overlapping sequences representing a putative bovine GGF-II gene and a cDNA.
Several PCR procedures were used to obtain additional coding sequences for the putative bovine GGF-II gene. Total RNA and oligo dT-selected (poly A containing) RNA samples were prepared from bovine total pituitary, anterior pituitary, posterior pituitary, and hypothalamus. Using primers from the list shown in Figure 23, one-sided PCR reactions (RACE) were used to amplify cDNA ends in both the 3' and 5' directions, and anchored PCR reactions were performed with degenerate oligonucleotide primers representing additional GGF-II peptides. Figure 24 summarizes the contiguous DNA structures and sequences obtained in those experiments. From the 3' RACE reactions, three alternatively spliced cDNA sequences were produced, which have been cloned and sequenced. A 5' RACE reaction led to the discovery of an additional exon containing coding sequence for at least 52 amino acids. Analysis of that deduced amino acid sequence revealed peptides GGF-II-6 and a sequence similar to GGF-I-18 (see below). The anchored PCR reactions led to the identification of (cDNA) coding sequences of peptides GGF-II-1, 2, 3 and 10 contained within an additional cDNA segment of 300 bp. Thus this clone contains nucleotide sequences encoding six out of the existing total of nine novel GGF-II peptide sequences.
The cloned gene was characterized first by constructing a physical map of GGF2BG1 that allowed us to position the coding sequences as they were found (see below, Figure 25). DNA probes from the coding sequences described above have been used to further identify DNA fragments containing the exons on this phage clone and to identify clones that overlap in both directions. The putative bovine GGF-II gene is divided into at least 5 exons, but only exons A and B have been mapped thus far. The summary of the contiguous coding sequences identified is shown in Figure 26. The exons are listed (alphabetically) in the order of their discovery. It is apparent from the intron/exon boundaries that exon B may be included in cDNAs that connect exon E and exon A.
That is, exon B cannot be spliced out without compromising the reading frame. Therefore, we suggest that three alternative splicing patterns can produce putative bovine GGF-II cDNA sequences 1, 2 and 3. The coding sequences of these, designated GGF2BPP1.CDS, GGF2BPP2.CDS and GGF2BPP3.CDS, respectively, are given in Figures 28a, 28b and 28c, respectively. The deduced amino acid sequence of the three cDNAs is also given in Figures 28a, 28b and 28c.
The three deduced structures encode proteins of lengths 207, 258 and 264 amino acids. The first 183 residues of the deduced protein sequence is identical in all three gene products. At position 184 the clones differ significantly. A codon for glycine GGT in GGF2BPP1 also serves as a splice donor for GGF2BPP2 and GGF2BPP3, which alternatively add on exons C and D or C, c/d and D, respectively. As the GGFIIbPP1 transcript ends adjacent to a canonical AATAAA polyadenylation sequence, we suggest that this truncated gene product represents a bona fide mature transcript. The other two longer gene products share the same 3' untranslated sequence and polyadenylation site. Interestingly, however, as exon c/d is 68 nucleotides in length, GGF2BPP2 and GGF2BPP3 encode two distinct C-terminal sequences from the same DNA sequence. That is, the final portion is encoded by two overlapping reading frames.

All three of these molecules contain six of the nine novel GGF-II peptide sequences (see Figure 12) and another peptide is highly homologous to GGF-I-18 (see Figure 27). This finding gives a high probability that this recombinant molecule encodes at least a portion of bovine GGF-II. Furthermore, the calculated isoelectric points for the three peptides are consistent with the physical properties of GGF-I and II. Since the molecular size of GGF-II is roughly 60 kd, the longest of the three cDNAs should encode a protein with nearly one-half of the predicted number of amino acids. Similar procedures to those described above can be used to obtain the remaining sequence in the full length clone.

EXAMPLE 5

GGF Splicing Variants

It is possible that the shorter gene product represented by splicing pattern 1 (see Figure 28a and Example 4) may bind the GGF receptor and lack Schwann cell mitogenic activity. Thus, it may represent a natural inhibitor of GGF-I and GGF-II. Recently, Chan et. al., Science 254:1382,(1991), have identified a competitive antagonist of hepatocyte growth factor encoded by an alternative transcript which produces a truncated molecule. Therefore, there is biological precedent for truncated growth factors acting as natural antagonists. Such a molecule might be therapeutically useful. For example, a natural inhibitor might be useful in treating a Schwannoma or other malignancies that are caused by overexpression of GGF-I and/or GGF-II. Preliminary hybridization and DNA sequence data indicate that some splicing variation may exist elsewhere in the gene; thus, further splicing variants of GGF may exist, putting the total number at more than three.

EXAMPLE 6

GGF Sequences in Various Species

Database searching has not revealed any meaningful similarities with known protein sequences. This suggests that GGF-II is the first member of a new family or superfamily of proteins. In cross hybridization studies (DNA blotting experiments) with other mammalian DNAs we have shown clearly that DNA probes from this bovine recombinant molecule can readily detect specific sequences in a variety of samples tested. A highly homologous sequence is also detected in human genomic DNA. The autoradiogram is shown in Figure 29. Furthermore, the blotting experiment shows that the probe detects bands of lesser intensity that might represent related genes, other potential members of a GGF family.

EXAMPLE 7

Cloning GGF-II Using Nucleotide Sequence Information

The gene encoding GGF-II may be readily cloned by those of skill in the art from a bovine genomic or pituitary cDNA library by using unique probes or primers identical to portions of the coding sequence disclosed herein. Furthermore, any vertebrate (including human) GGF-II may be cloned by using a genomic or pituitary library prepared from the appropriate species and using the approach described above (for the bovine sequence).

Thus, a cDNA library constructed using polyA RNA isolated from posterior pituitary can be plated (as described in Example 4) and plaque lifts probed with either radiolabelled fragments of GGF-II DNA (e.g. the 4 kb fragment released by a digest of cloned GGF2BG1 DNA with Eco RI). Alternatively a unique synthetic probe primer can be prepared from any GGF 2BG1 exon sequence, end labelled and used to screen a library as described above in Example 4. A third approach is to use unique PCR primer sets (prepared in a similar fashion to the probes described above) to amplify specific products from a variety of nucleic acid sources (e.g. poly A+ RNA, genomic or cDNA library DNA). These products can be subcloned and assembled to create a full length sequence encoding GGF-II.

EXAMPLE 8

Post-Translational Modification of GGF

The sequence of bovine GGF-II reveals several sites which can potentially be used by a cell to add carbohydrate to the protein following translation of the corresponding messenger RNA. The amino acid sequence X(P excluded) NXT/SX (P excluded) is a signal for the addition of an N linked carbohydrate moiety and can be potentially modified in the cell. Within the amino acid stretch from residues 1 to 183 (shared by the three deduced protein sequences) potential glycosylation sites occur four times, at residues 50, 123, 170, 176. A fifth site exists at residue 183 in GGF2BPP2.PEP and GGF2BPP3.PEP. This site is removed in GGF2BPP1.PEP by an alternative splicing event. The potential glycosylation sites at residues 50 and 123 are probably glycosylated in the GGF-II proteins(s). This conclusion is drawn from the peptide sequence data. An examination of the actual peptide sequences encompassing residues 50 and 123 (peptides GGF-II-2 and GGFI-18) reveals that the carbohydrate modifiable residue (N) in each sequence is not identifiable suggesting, in fact, that these residues are indeed glyosylated and are not detected in the sequencing process.

EXAMPLE 9

Preparation of Antisera Directed Against a Portion of GGF-II

Antibodies directed against GGF-II can be used to characterize GGF-II at the protein level. For this purpose a portion of the GGF-II sequence (nucleotides 281 to 653 (Figure 28a), which encode a 115 residue amino acid sequence has been expressed in a bacterial fusion protein expression system. The DNA fragment encoding this peptide was inserted in vector pMAL-C2 (New England Biolabs) and the resultant fusion gene was expressed and the protein was processed according to the manufacturers instructions.

The resultant fusion protein was eluted from the purification column, cleaved with the enzyme factor Xa (which releases the GGF-II portion of the peptide), and electrophoresed on a polyacrylamide gel, excised from the gel and used to immunize rabbits. Sera obtained from these rabbits following each boost can be tested by Western blot analysis (Burnette, Anal. Biochem. 112:195-203, 1983) of extracts prepared from bacteria harbouring the overproducing plasmids. This analysis can reveal whether antibodies have been produced that recognize the bacterially produced immunogen. The animals can be boosted until a significant positive response is achieved as determined in this assay. These antibodies may also be useful for diagnostic purposes.

EXAMPLE 10

Purification of GGFs From Recombinant Cells

In order to obtain full length or portions of GGFs to assay for biological activity, the proteins can be overproduced using cloned DNA. Several approaches can be used. A recombinant E. coli cell containing the sequences described above in Example 4 ("Results") can be constructed. Expression systems such as pNH8a or pHH16a (Stratagene, Inc) can be used for this purpose by following manufacturers procedures. Alternatively, these sequences can be inserted in a mammalian expression vector and an overproducing cell line can be constructed. As an example, for this purpose DNA encoding a GGF, with a homologous or a foreign secretion signal sequence attached, can be expressed in Chinese Hamster Ovary cells using the pMSXND expression vector (Lee and Nathans, J. Biol. Chem. 263: 3521-3527, 1981). This vector containing GGF DNA sequence can be transfected into CHO cells using established procedures.

G418-resistant clones can be grown in the presence of methotrexate to select for cells that amplify the dhfr gene (contained on the pMSXND vector) and, in the process, co-amplify the adjacent GGF protein encoding sequence. Because CHO cells can be maintained in a totally protein-free medium (Hamilton and Ham. In Vitro 13:537-547, 1977), the desired protein can be purified from the medium. Western analysis using the antisera produced in Example 8 can be used to detect the presence of the desired protein in the conditioned medium of the overproducing cells.

The desired protein can be purified from the E. coli lysate or the CHO cell conditioned medium using procedures described in Example 1.

The protein may be assayed at various points in the procedure using the Western blot assay described in Example 9. Alternatively, the Schwann cell mitogenic assay described herein may be used to assay the expressed product of the full length clone or any biologically active portions thereof.

**Claims**

1. A basic polypeptide factor or portion thereof obtainable by hydroxylapatite HPLC and having mitogenic activity stimulating the division of Schwann cells in the presence of foetal calf plasma, and when isolated using reversed-phase HPLC retaining at least 50% of said activity after 10 weeks incubation in 0.1% trifluoroacetic acid at 4°C, said basic polypeptide factor having a molecular weight of from 30 kD to 36 kD, and encoded by a DNA sequence shown in Figure 22.

2. A basic polypeptide factor or portion thereof obtainable by hydroxylapatite HPLC and stimulating the division of Schwann cells in the presence of foetal calf plasma, and when isolated using reversed-phase HPLC retaining at least 50% of said activity after 10 weeks incubation in 0.1% trifluoroacetic acid at 4°C, said basic polypeptide factor having a molecular weight of from 55 kD to 63 kD, and encoded by a DNA sequence as shown in any of Figures 28a, 28b or 28c; or the DNA sequence represented by nucleotides 281-557 of the sequence shown in figure 28a.

3. A process for the preparation of a polypeptide as claimed in claim 1 or claim 2 comprising extracting vertebrate brain material, optionally pituitary material, e.g. bovine pituitary to obtain protein, subjecting the resulting extract to chromatographic purification comprising hydroxylapatite HPLC and thereafter to SDS-polyacrylamide gel electrophoresis and collecting that fraction therefrom which has an observed molecular weight of 30 kD to 36 kD and/or that fraction which has an observed molecular weight of 55 kD to 63 kD if, in either case, subjected to SDS-polyacrylamide gel electrophoresis using the following molecular weight standards:

| | |
|---|---|
| Lysozyme (hen egg white) | 14,400 |
| Soybean trypsin inhibitor | 21,500 |
| Carbonic anhydrase (bovine) | 31,000 |
| Ovalbumin (hen egg white) | 45,000 |
| Bovine serum albumin | 66,200 |
| Phosphorylase B (rabbit muscle) | 97,400; |

in the case of the aforesaid smaller molecular weight fraction whether in reducing conditions or not, and in the case of the aforesaid larger molecular weight fraction under non-reducing conditions, and which fraction(s) exhibit(s) mitogenic activity stimulating the division of rat Schwann cells against a background of foetal calf plasma; optionally, the chromatographic purification including a step where in the protein initially extracted from brain material is first subjected to carboxymethyl cellulose chromatography and/or also including, after hydroxylapatite HPLC, cation exchange chromatography, gel filtration, and/or reversed-phase HPLC.

4. An isolated DNA having a sequence encoding a polypeptide having glial cell mitogenic activity and comprising:-

(a) a sequence shown in any one of Figures 28a, 28b or 28c;

(b) a sequence shown in Figure 22; or

(c) the sequence represented by nucleotides 281-557 of the sequence shown in Figure 28a.

5. A DNA construct comprising a DNA as defined in claim 4 and in operable reading frame position in a vector under the control of a control sequence permitting expression of said sequence in chosen host cells after transformation of said cells by said construct.

6. Host cells modified by incorporating a construct as defined in claim 5 so that DNA may be expressed in said host cells.

7. A process for the preparation of a glial cell mitogenic factor comprising cultivating modified host cells as defined in claim 6 under conditions permitting expression of said DNA sequence.

8. A pharmaceutical or veterinary formulation comprising a polypeptide as defined in claim 1 or claim 2 formulated for pharmaceutical or veterinary use, respectively, optionally together with an acceptable diluent, carrier or excipient and/or in unit dosage form.

9. A polypeptide as defined in claim 1 or claim 2 for use as a glial cell mitogen.

10. The use of a polypeptide as defined in claim 1 or claim 2

(a) for the manufacture of a medicament for the treatment of a nervous disease or disorder or for neural regeneration or repair; or

(b) as an immunogen to generate antibodies, optionally said antibodies being for diagnostic purposes.

11. The use of a polypeptide as defined in claim 1 or claim 2

   (a) in a competitive assay to identify or quantify molecules having receptor binding characteristics corresponding to those of said polypeptide, optionally said polypeptide being labelled, if desired with a radioisotope;

   (b) in an affinity isolation process, optionally affinity chromatography, for the separation of a corresponding receptor; or

   (c) as a glial cell mitogen in vitro.

12. The use of a polypeptide of claim 1 or claim 2, or a corresponding nucleic acid sequence, in investigating, isolating or preparing a glial cell mitogen or a gene sequence corresponding thereto, or in generating a corresponding antibody.

13. An antibody to a polypeptide as defined in claim 1 or claim 2.


**Patentansprüche**

1. Basischer Polypeptidfaktor oder Teil davon, der durch Hydroxylapatit-HPLC erhältlich ist und eine die Teilung von Schwann-Zellen in Gegenwart von fötalem Kälberplasma stimulierende mitogene Aktivität aufweist und, wenn er unter Verwendung einer Umkehrphasen-HPLC isoliert wird, mindestens 50 % dieser Aktivität nach 10 Wochen Inkubation in 0,1 % Trifluoressigsäure bei 4°C beibehält, wobei der basische Polypeptidfaktor ein Molekulargewicht von 30 kD bis 36 kD aufweist und von einer DNA-Sequenz kodiert wird, die in Fig. 22 gezeigt ist.

2. Basischer Polypeptidfaktor oder Teil davon, der durch Hydroxylapatit-HPLC erhältlich ist und die Teilung von Schwann-Zellen in Gegenwart von fötalem Kälberplasma stimuliert und, wenn er unter Verwendung einer Umkehrphasen-HPLC isoliert wird, mindestens 50 % dieser Aktivität nach 10 Wochen Inkubation in 0,1 % Trifluoressigsäure bei 4°C beibehält, wobei der basische Polypeptidfaktor ein Molekulargewicht von 55 kD bis 63 kD aufweist und von einer DNA-Sequenz, wie sie in einer der Fig. 28a, 28b oder 28c gezeigt ist; oder der DNA-Sequenz, die durch die Nukleotide 281-557 der in Fig. 28a gezeigten Sequenz wiedergegeben wird, kodiert wird.

3. Verfahren zum Herstellen eines Polypeptids nach Anspruch 1 oder Anspruch 2, umfassend das Extrahieren von Vertebraten-Hirnmaterial, gegebenenfalls Hypophysenmaterial, z.B. Rinderhypophyse, zum Erhalten von Protein, Unterziehen des resultierenden Extrakts einer chromatografischen Reinigung, umfassend Hydroxylapatit-HPLC, und danach einer SDS-Polyacrylamid-Gelelektrophorese und Sammeln der Fraktion davon, die ein beobachtetes Molekulargewicht von 30 kD bis 36 kD aufweist, und/oder der Fraktion, die ein beobachtetes Molekulargewicht von 55 kD bis 63 kD aufweist, wenn sie jeweils einer SDS-Polyacrylamid-Gelelektrophorese unter Verwendung der folgenden Molekulargewichtstandards unterzogen wird:

| | |
|---|---|
| Lysozym (Hühnereiweiß) | 14400 |
| Sojabohnen-Trypsin-Inhibitor | 21500 |
| Carboanhydrase (vom Rind) | 31000 |
| Ovalbumin (Hühnereiweiß) | 45000 |
| Rinderserumalbumin | 66200 |
| Phosphorylase B (Kaninchenmuskel) | 97400; |

und zwar im Fall der vorstehend erwähnten Fraktion mit kleinerem Molekulargewicht unter reduzierenden Bedingungen oder auch nicht und im Fall der vorstehend erwähnten Fraktion mit größerem Molekulargewicht unter nicht reduzierenden Bedingungen wobei die Fraktion(en) eine die Teilung von Ratten-Schwann-Zellen stimulierende mitogene Aktivität gegen einen Hintergrund von fötalem Kälberplasma aufweist bzw. aufweisen; wobei die chromatografische Reinigung gegebenenfalls einen Schritt einschließt, bei dem das anfänglich aus Himmaterial extrahierte Protein zuerst einer Carboxymethylcellulose-Chromatografie unterworfen wird, und/oder auch nach einer Hydroxylapatit-HPLC eine Kationenaustauschchromatografie, Gelfiltration und/oder Umkehrphasen-HPLC einschließt.

4. Isolierte DNA mit einer Sequenz, die ein Polypeptid mit mitogener Aktivität auf Gliazellen kodiert und umfasst:

(a) eine Sequenz, die in einer der Fig. 28a, 28b oder 28c gezeigt ist;

(b) eine Sequenz, die in Fig. 22 gezeigt ist; oder

(c) die Sequenz, die durch die Nukleotide 281-557 der in Fig. 28a gezeigten Sequenz wiedergegeben wird.

5.  DNA-Konstrukt, umfassend eine DNA wie in Anspruch 4 definiert und in funktioneller Anordnung mit dem Leseraster in einem Vektor unter der Kontrolle einer Kontrollsequenz, welche die Expression dieser Sequenz in ausgewählten Wirtszellen nach der Transformation dieser Zellen durch das Konstrukt ermöglicht.

6.  Wirtszellen, die durch den Einbau eines wie in Anspruch 5 definierten Konstrukts modifiziert sind, so dass DNA in diesen Wirtszellen exprimiert werden kann.

7.  Verfahren zum Herstellen eines für Gliazellen mitogenen Faktors, umfassend das Kultivieren von modifizierten Wirtszellen, wie in Anspruch 6 definiert, unter Bedingungen, welche die Expression dieser DNA-Sequenz ermöglichen.

8.  Pharmazeutische oder tiermedizinische Formulierung, umfassend ein Polypeptid wie in Anspruch 1 oder Anspruch 2 definiert, das für eine pharmazeutische oder tiermedizinische Anwendung formuliert ist, gegebenenfalls zusammen mit einem annehmbaren Verdünnungsmittel, Träger oder Excipiens und/oder in Einheitsdosisform.

9.  Polypeptid wie in Anspruch 1 oder Anspruch 2 definiert, zur Verwendung als Gliazellen-Mitogen.

10. Verwendung eines Polypeptids wie in Anspruch 1 oder Anspruch 2 definiert

(a) zum Herstellen eines Arzneimittels für die Behandlung einer Nervenkrankheit oder nervösen Störung oder für eine neurale Regeneration oder Reparatur; oder

(b) als Immunogen zum Erzeugen von Antikörpern, wobei diese Antikörper gegebenenfalls für diagnostische Zwecke bestimmt sind.

11. Verwendung eines Polypeptids wie in Anspruch 1 oder Anspruch 2 definiert

(a) in einem kompetitiven Assay zum Identifizieren oder Quantifizieren von Molekülen mit Rezeptorbindungseigenschaften, die denen des Polypeptids entsprechen, wobei das Polypeptid gegebenenfalls markiert ist, und, falls dies gewünscht wird, mit einem Radioisotop markiert ist;

(b) in einem Affinitätsisolierungsverfahren, gegebenenfalls einer Affinitätschromatografie, zur Abtrennung eines entsprechenden Rezeptors; oder

(c) als Gliazellen-Mitogen in vitro.

12. Verwendung eines Polypeptids nach Anspruch 1 oder Anspruch 2, oder einer entsprechenden Nukleinsäuresequenz, beim Untersuchen, Isolieren oder Herstellen eines Gliazellen-Mitogens oder einer diesem entsprechenden Gensequenz, oder beim Erzeugen eines entsprechenden Antikörpers.

13. Antikörper gegen ein Polypeptid, wie es in Anspruch 1 oder Anspruch 2 definiert ist.

**Revendications**

1.  Facteur polypeptidique basique ou portion de celui-ci, pouvant être obtenu par HPLC sur hydroxyapatite et possédant une activité mitogénique stimulant la division des cellules de Schwann en présence de plasma foetal bovin, et conservant, une fois isolé par HPLC en phase inverse, au moins 50% de ladite activité après 10 semaines d'incubation dans de l'acide trifluoroacétique à 0,1% à 4°C, ledit facteur polypeptidique basique ayant un poids moléculaire de 30 kD à 36 kD et étant codé par une séquence d'ADN présentée à la figure 22.

2.  Facteur polypeptidique basique ou portion de celui-ci, pouvant être obtenu par HPLC sur hydroxyapatite et possédant une activité mitogénique stimulant la division des cellules de Schwann en présence de plasma foetal bovin, et conservant, une fois isolé par HPLC en phase inverse, au moins 50% de ladite activité après 10 semaines d'incubation dans de l'acide trifluoroacétique à 0,1% à 4°C, ledit facteur polypeptidique basique ayant un poids moléculaire de 55 kD à 63 kD et étant codé par une séquence d'ADN telle que présentée à l'une quelconque des figures 28a, 28b ou 28c ; ou par la séquence d'ADN représentée par les nucléotides 281 à 557 de la séquence présentée à la figure 28a.

3. Procédé de préparation d'un polypeptide selon la revendication 1 ou la revendication 2 comprenant les étapes consistant à extraire le matériau cérébral d'un vertébré, éventuellement le matériau hypophysaire, par exemple l'hypophyse bovine, pour obtenir des protéines, à soumettre l'extrait résultant à une purification chromatographique, comprenant une HPLC sur hydroxyapatite, puis à une électrophorèse sur gel de polyacrylamide-SDS et à récupérer la fraction d'un poids moléculaire observé de 30 kD à 36 kD et/ou la fraction d'un poids moléculaire observé de 55 à 63 kD, determiné dans tous les cas par électrophorèse sur un gel de polyacrylamide-SDS en utilisant les étalons de poids moléculaire suivants :

| | |
|---|---|
| Lysozyme (blanc d'oeuf de poule) | 14.400 |
| Inhibiteur de la trypsine de soja | 21.500 |
| Anhydrase carbonique (bovine) | 31.000 |
| Ovalbumine (blanc d'oeuf de poule) | 45.000 |
| Albumine de sérum de boeuf | 66.200 |
| Phosphorylase B (muscle de lapin) | 97.400 ; |

dans des conditions réductrices ou non dans le cas de la fraction susmentionnée de plus petit poids moléculaire et dans le cas de la fraction susmentionnée de plus grand poids moléculaire, dans des conditions non réductrices, la ou lesquelles fractions présentant une activité mitogénique stimulant la division de cellules de Schwann de rat par rapport à un contrôle de plasma foetal bovin ; la purification chromatographique incluant éventuellement une étape dans laquelle la protéine initialement extraite à partir du matériau cérébral est d'abord soumise à une chromatographie sur de la carboxyméthylcellulose et/ou incluant également, après l'HPLC sur hydroxyapatite une chromatographie à échange de cations, une gel-filtration et/ou une HPLC en phase inverse.

4. ADN isolé possédant une séquence codant pour un polypeptide possédant une activité mitogénique sur les cellules gliales et comprenant :

   a) une séquence montrée à l'une quelconque des figures 28a, 28b ou 28c ;
   b) une séquence montrée à la figure 22 ; ou
   c) la séquence représentée par les nucléotides 281-557 de la séquence montrée à la figure 28a.

5. Une construction d'ADN comprenant un ADN tel que défini à la revendication 4 dans un cadre de lecture opérationnel dans un vecteur sous le contrôle d'une séquence de régulation permettant l'expression de ladite séquence dans des cellules hôtes choisies après transformation desdites cellules par ladite construction.

6. Cellules hôtes modifiées par l'incorporation d'une construction telle que définie à la revendication 5 de telle sorte que l'ADN puisse être exprimé dans lesdites cellules hôtes.

7. Procédé de préparation d'un facteur mitogénique des cellules gliales comprenant la culture des cellules hôtes modifiées telles que définies à la revendication 6 dans des conditions permettant l'expression de ladite séquence d'ADN.

8. Formulation pharmaceutique ou vétérinaire comprenant un polypeptide tel que défini à la revendication 1 ou à la revendication 2 formulé pour un usage pharmaceutique ou vétérinaire, respectivement, éventuellement avec un diluant, un support ou un excipient acceptable et/ou sous forme de dosage unitaire.

9. Polypeptide tel que défini à la revendication 1 ou la revendication 2 destiné à être utilisé comme mitogène des cellules gliales.

10. Utilisation d'un polypeptide tel que défini à la revendication 1 ou à la revendication 2 :

(a) pour la fabrication d'un médicament destiné au traitement d'une maladie ou d'un trouble du système nerveux, ou à la régénération ou à la réparation neuronale ; ou

(b) en tant qu'immunogène pour la préparation d'anticorps, lesdits anticorps étant éventuellement destinés à des fins de diagnostic.

11. Utilisation d'un polypeptide tel que défini à la revendication 1 ou à la revendication 2

(a) dans un test compétitif destiné à identifier ou à doser des molécules possédant des caractéristiques de liaison à un récepteur correspondant à celles dudit polypeptide, ledit polypeptide étant éventuellement marqué, si on le souhaite, avec un isotope radioactif ;

(b) dans un procédé d'isolement par affinité, éventuellement de chromatographie par affinité, pour la séparation d'un récepteur correspondant ; ou

(c) en tant que mitogène des cellules gliales in vitro.

12. Utilisation d'un polypeptide selon la revendication 1 ou la revendication 2, ou d'une séquence d'acide nucléique correspondante pour étudier, isoler ou préparer un mitogène des cellules gliales ou une séquence génétique correspondante, ou pour produire un anticorps correspondant.

13. Anticorps dirigé contre un polypeptide tel que défini à la revendication 1 ou à la revendication 2.

FIGURE 1

FIGURE 2

FIGURE 3

FIGURE 4

FIGURE 5

FIGURE 6

FIGURE 7

FIGURE 8

**N-terminus**

GGF-I 01      F K G D A H T E

**Trypsin peptides**

| | | |
|---|---|---|
| GGF-I 02 | K/R A S L A D E Y E Y M X K * | |
| GGF-I 03 | K/R T E T S S S G L X L K * | |
| GGF-I 04 | K/R K L G E M W A E | HMG-1 |
| GGF-I 05 | K/R L G E K R A | HMG-1? |
| GGF-I 06 | K/R I·K S E H A G L S I G D T A K * | HMG-2 |
| GGF-I 07 | K/R A S L A D E Y E Y M R K * | |
| GGF-I 08 | K/R I K G E H P G L S I G D V A K * | HMG-1 |
| GGF-I 09 | K/R M S E Y A F F V Q T X R * | HMG-2 |
| GGF-I 10 | K/R S E H P G L S I G D T A K * | HMG-1 |
| GGF-I 11 | K/R A G Y F A E X A R * | |
| GGF-I 12 | K/R K L E F L X A K * | |
| GGF-I 13 | K/R T T E M A S E Q G A | |
| GGF-I 14 | K/R A K E A L A A L K * | |
| GGF-I 15 | K/R F V L Q A K K * | |
| GGF-I 16 | K/R L G E M W | HMG-1 |

**Protease V8 peptides**

| | | |
|---|---|---|
| GGF-I 17 | E T Q P D P G Q I L K K V P M V I G A Y T | |
| GGF-I 18 | E Y K C L K F K W F K K A T V M | |
| GGF-I 19 | E A K Y F S K X D A | LH-alpha |
| GGF-I 20 | E X K F Y V P | |
| GGF-I 21 | E L S F A S V R L P G C P P G V D P M V S F P V A L | LH-beta |

FIGURE 9

34

EP 0 579 640 B1

A

GGF-I 01    F K G D A H T E

GGF-I 02    A S L A D E Y E Y M X K

GGF-I 03    T E T S S S G L X L K

GGF-I 07    A S L A D E Y E Y M R K

GGF-I 11    A G Y F A E X A R

GGF-I 13    T T E M A S E Q G A

GGF-I 14    A K E A L A A L K

GGF-I 15    F V L Q A K K

GGF-I 17    E T Q P D P G Q I L K K V P M V I G A Y T

GGF-I 18    E Y K C L K F K W F K K A T V M

B

GGF-I 20    E X K F Y V P

GGF-I 12    K L E F L X A K

FIGURE 10

EP 0 579 640 B1

FIGURE 11

36

**Trypsin peptides**

GGF-II 01   K/R  V  H  Q  V  W  A  A  K  *

GGF-II 02   K/R  Y  I  F  F  M  E  P  E  A  X  S  S  G

GGF-II 03   K/R  L  G  A  W  G  P  P  A  F  P  V  X  Y

GGF-II 04   K/R  W  F  V  V  I  E  G  K  *

GGF-II 05   K/R  A  L  A  A  A  G  Y  D  V  E  K  *

GGF-II 06   K/R  L  V  L  R  *

GGF-II 07   K/R  X  X  Y  P  G  Q  I  T  S  N

GGF-II 08   K/R  A  S  P  V  S  V  G  S  V  Q  E  L  V  Q  R  *

GGF-II 09   K/R  V  C  L  L  T  V  A  A  P  P  T

GGF-II 10   K/R  D  L  L  L  X  V

Histone H1

Trypsin

**Lysyl Endopeptidase-C peptides**

GGF-II 11   K  V  H  Q  V  W  A  A  K  *

GGF-II 12   K  A  S  L  A  D  S  G  E  Y  M  X  K  *

A

GGF-II 01  V H Q V W A A K

GGF-II 02  Y I F F M E P E A X S S G

GGF-II 03  L G A W G P P A F P V X Y

GGF-II 04  W F V V I E G K

GGF-II 08  A S P V S V G S V Q E L V Q R

GGF-II 09  V C L L T V A A P P T

GGF-II 11  K V H Q V W A A K

GGF-II 12  K A S L A D S G E Y M X K

B  Novel Factor II Peptides – others

GGF-II 10  D L L L X V

FIGURE 12

FIGURE 13

FIGURE 14A

FIGURE 14B

FIGURE 15

FIGURE 16

FIGURE 17

FIGURE 18

FIGURE 19

FIGURE 20

| Oligo | Sequence | Peptide |
|-------|----------|---------|
| 535 | TTYAARGGNGAYGCNCAYAC! | GGFI-1 |
| 536 | CATRTAYTCRTAYTCRTCNGC! | GGFI-2 |
| 537 | TGYTCNGANGCCATYTCNGT! | GGFI-13 |
| 538 | TGYTCRCTNGCCATYTCNGT!' | GGFI-13 |
| 539 | CCDATNACCATNGGNACYTT! | GGFI-17 |
| 540 | GCNGCCCANACYTGRTGNAC! | GGFII-1 |
| 541 | GCYTCNGGYTCCATRAARAA! | GGFII-2 |
| 542 | CCYTCDATNACNACRAACCA! | GGFII-4 |
| 543 | TCNGCRAARTANCCNGC! | GGFI-11 |
| 544 | GCNGCNAGNGCYTCYTTNGC! | GGFI-14 |
| 545 | GCNGCYAANGCYTCYTTNGC! | GGFI-14 |
| 546 | TTYTTNGCYTGNAGNACRAA! | GGFI-15 |
| 551 | TTYTTNGCYTGYAANACRAA! | GGFI-15 |
| 568 | TGNACNAGYTCYTGNAC! | GGFII-8 |
| 569 | TGNACYAAYTCYTGNAC! | GGFII-8 |
| 609 | CATRTAYTCNCCNGARTCNGC! | GGFII-12 |
| 610 | CATRTAYTCNCCRCTRTCNGC! | GGFII-12 |
| 649 | NGARTCNGCYAANGANGCYTT! | GGFII-12 |
| 650 | NGARTCNGCNAGNGANGCYTT! | GGFII-12 |
| 651 | RCTRTCNGCYAANGANGCYTT! | GGFII-12 |
| 652 | RCTRTCNGCNAGNGANGCYTT! | GGFII-12 |
| 653 | NGARTCNGCYAARCTNGCYTT! | GGFII-12 |
| 654 | NGARTCNGCNAGRCTNGCYTT! | GGFII-12 |
| 655 | RCTRTCNGCYAARCTNGCYTT! | GGFII-12 |
| 656 | RCTRCTNGCNAGRCTNGCYTT! | GGFII-12 |
| 659 | ACNACNGARATGGCTCNNGA! | GGFI-13 |
| 660 | ACNACNGARATGGCAGYNGA! | GGFI-13 |
| 661 | CAYCARGTNTGGGCNGCNAA! | GGFII-1 |
| 662 | TTYGTNGTNATHGARGGNAA! | GGFII-4 |
| 663 | AARGGNGAYGCNCAYACNGA! | GGFI-1 |
| 664 | GARGCNYTNGCNGCNYTNAA! | GGFI-14 |
| 665 | GTNGGNTCNGTNCARGARYT! | GGFII-8 |
| 666 | GTNGGNAGYGTNCARGARYT! | GGFII-8 |
| 694 | NACYTTYTTNARHATYTGNCC! | GGFI-17 |

FIGURE 21

47

EP 0 579 640 B1

TCTAAAACTACAGAGACTGTATTTTCATGATCATCATAGTTCTGTGAAATATACTTAAACCGCTTTGGTCCTGATCTTGTAGGAAGTCAGAACTTCGCAT 100
\* N Y R D C I F M I I I V L \* N I L K P L W S \* S C R K S E L R I

TAGCAAAGCGTCACTGGCTGATTCTGGAGAATATATGTGCAAAGTGATCAGCAAACTAGGAAATGACAGTGCCTCTGCCAACATCACCATTGTGGAGTCA 200
S K A S L A D S G E Y M C K V I S K L G N D S A S A N I T I V E S

AACGGTAAGAGATGCCTACTGCGTGCTATTTCTCAGTCTCTAAGAGGAGTGATCAAGGTATGTGGTCACACTTGAATCACGCAGGTGTGTGAAATCTCAT 300
N G K R C L L R A I S Q S L R G V I K V C G H T \* I T Q V C E I S L

TGTGAACAAATAAAAATCATGAAAGGAAAACTCTATGTTTGAAATATCTTATGGGTCCTCCTGTAAAGCTCTTCACTCCATAAGGTGAAATAGACCTGAA 400
\* T N K N H E R K T L C L K Y L M G P P V K L F T P \* G E I D L K

ATATATATAGATTATTT
Y I \* I I

FIGURE 22

Degenerate PCR primers

| Oligo | Sequence | Peptide |
|-------|----------|---------|
| 657 | CCGAATTCTGCAGGARACNCARCCNGAYCCNGG! | GGFI-17 |
| 658 | AAGGATCCTGCAGNGTRTANGCNCCHATNACCATNGG! | GGFI-17 |
| 667 | CCGAATTCTGCAGGCNGAYTCNGGNGARTAYATG! | GGFII-12 |
| 668 | CCGAATTCTGCAGGCNGAYATYGGNGARTAYAT! | GGFII-12 |
| 669 | AAGGATCCTGCAGNNNCATRTAYTCNCCNGARTC! | GGFII-12 |
| 670 | AAGGATCCTGCAGNNNCATRTAYTCNCCRRTRTC! | GGFII-12 |
| 671 | CCGAATTCTGCAGCAYCARGTNTGGGCNGCNAA! | GGFII-1 |
| 672 | CCGAATTCTGCAGATHTTYTTYATGGARCCNGARG! | GGFII-2 |
| 673 | CCGAATTCTGCAGGGGGNCCNCCNGCNTTYCCNGT! | GGFII-3 |
| 674 | CCGAATTCTGCAGTGGTTYGTNGTNATHGARGG! | GGFII-4 |
| 677 | AAGGATCCTGCAGYTTNGCNGCCCANACYTGRTG! | GGFII-1 |
| 678 | AAGGATCCTGCAGGCYTCNGGYTCCATRAARAA! | GGFII-2 |
| 679 | AAGGATCCTGCAGACNGGRAANGCNGGNGGNCC! | GGFII-3 |
| 680 | AAGGATCCTGCAGYTTNCCYTCDATNACNACRAAC! | GGFII-4 |
| 681 | CATRTAYTCRTAYTCTCNGCAAGGATCCTGCAG! | GGFI-2 |
| 682 | CCGAATTCTGCAGAARGGNGAYGCNCAYACNGA! | GGFI-1 |
| 683 | GCNGCYAANGCYRCYTTNGCAAGGATCCTGCAG! | GGFI-14 |
| 684 | GCNGCNAGNGCYTCYTTNGCAAGGATCCTGCAG! | GGFI-14 |
| 685 | TCNGCRAARTANCCNGCAAGGATCCTGCAG! | GGFII-1 |

Unique PCR primers for Factor II

| Oligo | Sequence | Comment |
|-------|----------|---------|
| 711 | CATCGATCTGCAGGCTGATTCTGGAGAATATATGTGCA! | 3' RACE |
| 712 | AAGGATCCTGCAGCCACATCTCGAGTCGACATCGATT! | 3' RACE |
| 713 | CCGAATTCTGCAGTGATCAGCAAACTAGGAAATGACA! | 3' RACE |
| 721 | CATCGATCTGCAGCCTAGTTTGCTGATCACTTTGCAC! | 5' RACE |
| 722 | AAGGATCCTGCAGTATATTCTCCAGAATCAGCCAGTG! | 5' RACE; ANCHORED |
| 725 | AAGGATCCTGCAGGCACGCAGTAGGCATCTCTTA! | EXON A |
| 726 | CCGAATTCTGCAGCAGAACTTCGCATTAGCAAAGC! | EXON A |
| 771 | CATCCCGGGATGAAGAGTCAGGAGTCTGTGGCA! | EXONS B+A |
| 772 | ATACCCGGGCTGCAGACAATGAGATTTCACACACCTGCG! | |
| 773 | AAGGATCCTGCAGTTTGGAACCTGCCACAGACTCCT! | ANCHORED |
| 776 | ATACCCGGGCTGCAGATGAGATTTCACACACCTGCGTGA! | EXONS B+A |

FIGURE 23

exon e        exon b        exon a           exon c    exon c/d    exon d

p 711/713

p 487/712

p 711/713

p487/712

p 711/713

p487/712

p 771

p 487              p 776

p 722

p 671

p 672      p 722

p 722

p 671

p 773

p 672

p 773

FIGURE 24

50

FIGURE 25

EP 0 579 640 B1

FIGURE 26

EXONS

E  B  A  C  C/D  D

1. GGF2BPP1

2. GGF2BPP2

3. GGF2BPP3

EP 0 579 640 B1

52

```
Peptide        Pos.       Sequence match

II-1                      VHQVWAAK
                 1:        HQVWAAK AAGLK


II-10                     DLLLXV
                14: GGLKK dslltv RLGAW


II-03                     LGAWGPPAFPVXY
                21: LLTVR lgawghpafpscg RLKED


II-02                     YIFFMEPEAXSSG
                41: KEDSR YIFFMEPEANSSG GPGRL


II-6                      LVLR
               103: VAGSK LVLR CETSS


I-18                      EYKCLKFKWFKKATVM
               112: CETSS eysslkfkwfkngsel SRKNK


II-12                     KASLADSGEYMXK
               151: ELRIS KASLADSGEYMCK VISKL


I-07                      ASLADEYEYMRK
               152: LRISK asladsgeymck VISKL
```

FIGURE 27

```
CCTGCAGCATcArGTGTGGgCGGCgAAAGCcgGGGGGCTTGAAGAAgGACTCGCTGCTCACCGTgCgcCTGGGCgCcTGGgGCCACCCCGCCTTCCCCTCc
  H  Q  V  W  A  A  K  A  G  G  L  K  K  D  S  L  L  T  V  R  L  G  A  W  G  H  P  A  F  P  S
                                                                                          100


TgCGGGCGCCTCAAGGAGGACAGCAGGTACATCTTCTTCATGGAgcCCGAGGCCAACAGCAgcggCgGgcCcggccgccTtCcGAGCCTcCtTcCCCCCT
C  G  R  L  K  E  D  S  R  Y  I  F  F  M  E  P  E  A  N̲  S̲  S̲  G  G  P  G  R  L  P  S  L  L  P  P  S
                                                                                          200


CTCGAGACGGGCCGGAACCTcAAGAAGGAGgTCAGCCGGGTGCTGTGCAACgGTGCGCCTTGCCTCCCCGcTTGAAaGAGATGAAGAGTCAGGAGTCTGT
  R  D  G  P  E  P  Q  E  G  G  Q  P  G  A  V  Q  R  C  A  L  P  P  R  L  K  E  M  K  S  Q  E  S  V
                                                                                          300


GGCAGGTTCCAAACTAGTGCTTCGGTGCGAGACCAGTTCTGAATACTCCTCTCTCAAGTTCAAgTGGtTCAAgAATGGGAGTGaaTTAAGCCGAAAGAAc
A  G  S  K  L  V  L  R  C  E  T  S  S  E  Y  S  S  L  K  F  K  W  F  K  N̲  G  S̲  E  L  S  R  K  N
                                                                                          400


AAACCAgAAAACATCAAGATACAGAAAAGGCCGGGGAAGTCAGAACTTCGCATTAGCAAAGCGTCACTGGCTGATTCTGGAGAATATATGTGCAAAGTGA
K  P  E  N  I  K  I  Q  K  R  P  G  K  S  E  L  R  I  S  K  A  S  L  A  D  S  G  E  Y  M  C  K  V  I
                                                                                          500


TCAGCAAACTAGGAAATGACAGTGCCTCTGCCAACATCACCATTGTGGAGTCAAACGGTAAGAGATGCCTACTGCGTGCTATTTCTCAGTCTCTAAGAGG
  S  K  L  G  N̲  D  S̲  A  S  A  N̲  I  T̲  I  V  E  S  N  G  K  R  C  L  L  R  A  I  S  Q  S  L  R  G
                                                                                          600


AGTGATCAAGGTATGTGGTCACACTTGAATCACGCAGGTGTGTGAAATCTCATTGTGAACAAATAAAAATCATGAAAGGAAAAAAAAAAAAAAAAAAATCG
V  I  K  V  C  G  H  T  *
                                                                                          700


ATGTCGACTCGAGATGTGGCTGCAGGTCGACTCTAGAGGATCCC
```

FIGURE 28A

```
CCTGCAGCATcArGTGTGGgCGGCgAAAGCcgGGGGCTTGAAGAAgGACTCGCTGCTCACCGTgCgcCTGGGCgCcTGGgGCCACCCCGCCTTCCCCTCc
   H  Q  V  W  A  A  K  A  G  G  L  K  K  D  S  L  L  T  V  R  L  G  A  W  G  H  P  A  F  P  S
                                                                                            100

TgCGGGCGCCTCAAGGAGGACAGCAGGTACATCTTCTTCATGGAgcCCGAGGCCAACAGCAgcggCgGgcCcggccgccTtCcGAGCCTcCtTcCCCCCT
 C  G  R  L  K  E  D  S  R  Y  I  F  F  M  E  P  E  A  N  S  S  G  G  P  G  R  L  P  S  L  L  P  P  S
                                                                                            200

CTCGAGACGGGCCGGAACCTcAAGAAGGAGgTCAGCCGGGTGCTGTGCAACgGTGCGCCTTGCCTCCCCGcTTGAAaGAGATGAAGAGTCAGGAGTCTGT
  R  D  G  P  E  P  Q  E  G  G  Q  P  G  A  V  Q  R  C  A  L  P  P  R  L  K  E  M  K  S  Q  E  S  V
                                                                                            300

GGCAGGTTCCAAACTAGTGCTTCGGTGCGAGACCAGTTCTGAATACTCCTCTCTCAAGTTCAAgTGGtTCAAgAATGGGAGTGaaTTAAGCCGAAAGAAc
 A  G  S  K  L  V  L  R  C  E  T  S  S  E  Y  S  S  L  K  F  K  W  F  K  N  G  S  E  L  S  R  K  N
                                                                                            400

AAACCAgAAAACATCAAGATACAGAAAAGGCCGGGGAAGTCAGAACTTCGCATTAGCAAAGCGTCACTGGCTGATTCTGGAGAATATATGTGCAAAGTGA
 K  P  E  N  I  K  I  Q  K  R  P  G  K  S  E  L  R  I  S  K  A  S  L  A  D  S  G  E  Y  M  C  K  V  I
                                                                                            500

TCAGCAAACTAGGAAATGACAGTGCCTCTGCCAACATCACCATTGTGGAGTCAAACGCCACATCCACATCTACAGCTGGGACAAGCCATCTTGTCAAGTG
  S  K  L  G  N  D  S  A  S  A  N  I  T  I  V  E  S  N  A  T  S  T  S  T  A  G  T  S  H  L  V  K  C
                                                                                            600

TGCAGAGAAGGAGAAAACTTTCTGTGTGAATGGAGGCGAGTGCTTCATGGTGAAAGACCTTTCAAATCCCTCAAGATACTTGTGCAAGTGCCAACCTGGA
 A  E  K  E  K  T  F  C  V  N  G  G  E  C  F  M  V  K  D  L  S  N  P  S  R  Y  L  C  K  C  Q  P  G
                                                                                            700

TTCACTGGAGCGAGATGTACTGAGAATGTGCCCATGAAAGTCCAAACCCAAGAAAGTGCCCAAATGAGTTTACTGGTGATCGCTGCCAAAACTACGTAAT
 F  T  G  A  R  C  T  E  N  V  P  M  K  V  Q  T  Q  E  S  A  Q  M  S  L  L  V  I  A  A  K  T  T  *
                                                                                            800
```

FIGURE 28B
(PART)

EP 0 579 640 B1

GGCCAGCTTCTACAGTACGTCCACTCCCTTTCTGTCTCTGCCTGAATAGCGCATCTCAGTCGGTGCCGCtTTCTTGTTGCCGCATCTCCCCTCAGATTCC
900

rcctAGAGCTAGATGCGTTTTACCAGGTCTAACATTGACTGCCTCTGCCTGTCGCATGAGAACATTAACACAAGCGATTGTATGACTTCCTCTGTCCGTG
1000

ACTAGTGGGCTCTGAGCTACTCGTAGGTGCGTAAGGCTCCAGTGTTTCTGAAATTGATCtTGAATTACTGTGATaCGACATGATAGTCCCTCTCACCCAG
1100

TGCAATGACAATAAAGGCCTTGAAAAGTCAAAAAAAAAAAAAAAAAAAAAAAATCGATGTCGACTCGAGATGTGGCTGCAGGTCGACTCTAGAG

56

FIGURE 28B
(CONT'D)

CCTGCAGCATcArGTGTGGgCGGCgAAAGCcgGGGGCTTGAAGAAgGACTCGCTGCTCACCGTgCgcCTGGGCgCcTGGgGCCACCCCGCCTTCCCCTcc
H Q V W A A K A G G L K K D S L L T V R L G A W G H P A F P S
100

TgCGGGCGCCTCAAGGAGGACAGCAGGTACATCTTCTTCATGGAgcCCGAGGCCAACAGCAgcggCgGgcCcggccgccTtCcGAGCCTcCtTcCCCCCT
C G R L K E D S R Y I F F M E P E A N S S G G P G R L P S L L P P S
200

CTCGAGACGGGCCGGAACCTcAAGAAGGAGgTCAGCCGGGTGCTGTGCAACgGTGCGCCTTGCCTCCCCGcTTGAAaGAGATGAAGAGTCAGGAGTCTGT
R D G P E P Q E G G Q P G A V Q R C A L P P R L K E M K S Q E S V
300

GGCAGGTTCCAAACTAGTGCTTCGGTGCGAGACCAGTTCTGAATACTCCTCTCTCAAGTTCAAgTGGtTCAAgAATGGGAGTGaaTTAAGCCGAAAGAAc
A G S K L V L R C E T S S E Y S S L K F K W F K N G S E L S R K N
400

AAACCAgAAAACATCAAGATACAGAAAAGGCCGGGGAAGTCAGAACTTCGCATTAGCAAAGCGTCACTGGCTGATTCTGGAGAATATATGTGCAAAGTGA
K P E N I K I Q K R P G K S E L R I S K A S L A D S G E Y M C K V I
500

TCAGCAAACTAGGAAATGACAGTGCCTCTGCCAACATCACCATTGTGGAGTCAAACGCCACATCCACATCTACAGCTgGgACAAGCCATCTTGTCAAGTG
S K L G N D S A S A N I T I V E S N A T S T S T A G T S H L V K C
600

TGCAGAGAAGGAGAAAACTTTCTGTGTGAATGGAGGCGAgTGCTTCATGGTGAAAgACCTTTCAAATCCCTCAAGATACTTGTGCAAGTGCCCAAATGAG
A E K E K T F C V N G G E C F M V K D L S N P S R Y L C K C P N E
700

TTTACTGGTGATCGCTGCCAAAACTACGTAATGGCCAGCTTCTACAGTACGTCCACTCCCTTTCTGTCTCTGCCTGAATAGCGCATcTCAGTCGGTGCCG
F T G D R C Q N Y V M A S F Y S T S T P F L S L P E *
800

FIGURE 28C
(PART)

EP 0 579 640 B1

EP 0 579 640 B1

CTTTCTTGTTgCCGGCATCTCCCCTCAGATTCCGCCTAGAGCTAGATGCGTTTTACCAGGTCTAACATTGACTGCCTGCCTCTGCCTGTCGCATGAGAACATTAA 900

CACAAGCCGATTGTATGACTTCCTCTGTCCGTGACTAGTGGGCTCTGAGCTACTCGTAGGTGCGTAAGGCTCCAGTGTTTCTGAAATTGATCTTGAATTAC 1000

TGTGATACGACATGATAGTCCCCTCTCACCCAGTGCAATGACAATAAAGGCCTTGAAAAGTCAAAAAAAAAAAAAAAATCGATGTCGACTCGAGA 1100

TGTGGCTG

FIGURE 28C
(CONT'D)

58

FIGURE 29